# EUROPEAN PATENT SPECIFICATION

(11) **EP 1 689 871 B1**
(45) Date of publication and mention of the grant of the patent: **22.04.2009**
(21) Application number: 04803501.8
(22) Date of filing: 03.12.2004
(51) Int. Cl.: C12N 15/82, C12N 5/10

(54) **CONTROLLING GENE EXPRESSION IN PLASTIDS**
KONTROLLE DER GENEXPRESSION IN PLASTIDEN
CONTROLE DE L'EXPRESSION DE GENE DANS DES PLASTES

(30) Priority: 03.12.2003 WO PCT/EP03/13656
(43) Date of publication of application: 16.08.2006
(73) Proprietor: Icon Genetics GmbH, 80333 München (DE)
(72) Inventor: MÜHLBAUER, Stefan, 85345 Freising (DE)
(74) Representative: Blodig, Wolfgang
(86) International application number: PCT/EP2004/013780
(87) International publication number: WO 2005/054481

(56) References cited:
- WO-A-01/02593
- WO-A-98/11235
- US-A- 5 877 402
- US-A- 5 925 806
- US-A1- 2003 009 783
- HEIFETZ P B ET AL: "TRANSGENICS AND BIOTECHNOLOGY CHEMICAL REGULATION OF NUCLEAR AND PLASTID TRANSGENES IN PLANTS" PLANT PHYSIOLOGY, AMERICAN SOCIETY OF PLANT PHYSIOLOGISTS, ROCKVILLE, MD, US, vol. 114, no. 3, 5 August 1997 (1997-08-05), page 308, XP002058230 ISSN: 0032-0889 cited in the application
- HEIFETZ P B: "Genetic engeneering of the chloroplast" BIOCHIMIE, MASSON, PARIS, FR, vol. 82, 2000, pages 655-666, XP002190805 ISSN: 0300-9084
- THOMPSON R J ET AL: "STIMULATION OF A CHLAMYDOMONAS CHLOROPLAST PROMOTER BY NOVOBIOCIN IN-SITU AND IN ESCHERICHIA-COLI IMPLIES REGULATION BY TORSIONAL STRESS IN THE CHLOROPLAST DNA" CELL, vol. 48, no. 2, 1987, pages 281-288, XP009027675 ISSN: 0092-8674

### SEQUENCE LISTING

<110> Icon Genetics AG
   Muehlbauer, Stefan
<120> Controlling Gene Expression in Plastids
<130> PCT-13034
<140> PCT/EP2004/013780
   <141> 2004-12-03
<150> PCT/EP03/13656
   <151> 2003-12-03
<160> 21
<170> PatentIn version 3.1
<210> 1
   <211> 29
   <212> DNA
   <213> Artificial Sequence
<220>
   <223> PCR primer
<400> 1
   gaccatggaa ccagtaacgt tatacgatg 29
<210> 2
   <211> 26
   <212> DNA
   <213> Artificial Sequence
<220>
   <223> PCR primer
<400> 2
   cactgcagtc actgcccgct ttccag 26
<210> 3
   <211> 40
   <212> DNA
   <213> Artificial Sequence
<220>
   <223> PCR primer
<400> 3
   acgattgtga gcggataaca atatatttct gggagcgaac 40
<210> 4
   <211> 21
   <212> DNA
   <213> Artificial Sequence
<220>
   <223> PCR primer
<400> 4
   caatcccacg agcctcttat c 21
<210> 5
   <211> 29
   <212> DNA
   <213> Artificial Sequence
<220>
   <223> PCR primer
<400> 5
   gaccatggct agattagata aaagtaaag 29
<210> 6
   <211> 32
   <212> DNA .
   <213> Artificial Sequence
<220>
   <223> PCR primer
<400> 6
   cactgcagtt aagacccact ttcacattta ag 32
<210> 7
   <211> 40
   <212> DNA
   <213> Artificial Sequence
<220>
   <223> PCR primer
<400> 7
   acgtccctat cagtgataga gtatatttct gggagcgaac 40
<210> 8
   <211> 21
   <212> DNA
   <213> Artificial Sequence
<220>
   <223> PCR primer
<400> 8
   caatcccacg agcctcttat c 21
<210> 9
   <211> 36
   <212> DNA
   <213> Artificial Sequence
<220>
   <223> riboswitch
<400> 9
   agatgatacc agccgaaagg cccttggcag ctctcg 36
<210> 10
   <211> 78
   <212> DNA
   <213> Artificial Sequence
<220>
   <223> PCR primer
<400> 10
   tttggatccg aattctacca ttcacctctt ggatttgggt attaaagagg agaaggtata 60
   tgagtaaagg agaagaac 78
<210> 11
   <211> 33
   <212> DNA
   <213> Artificial Sequence
<220>
   <223> PCR primer
<400> 11
   tatgagctct tatttgtata gttcatccat gcc 33
<210> 12
   <211> 89
   <212> DNA
   <213> Artificial Sequence
<220>
   <223> cloning oligo
<400> 12
   tttcggccgc cgtcgttcaa tgagaatgga taagaggctc gtgggattga cgattgtgag 60
   cggataacaa tatatttctg ggagcgaac 89
<210> 13
   <211> 110
   <212> DNA
   <213> Artificial Sequence
<220>
   <223> cloning oligo
<400> 13
   tttcggccgt ctagagatat atggtagtag taagttaatt ttcattaacc accactacca 60
   atcacctcct ggatttgggt cgcccggagt tcgctcccag aaatatattg 110
<210> 14
   <211> 113
   <212> DNA
   <213> Artificial Sequence
<220>
   <223> cloning oligo
<400> 14
   tttcggccgt cgttcaatga gaatggataa gaggctcgtg ggattgacgt gagggggcag      60
   ggatggctat atttctggga gcgaacggaa atgctagcca tatgtatatc tcc            113
<210> 15
   <211> 116
   <212> DNA
   <213> Artificial Sequence
<220>
   <223> cloning oligo
<400> 15
   tttcggccgc cgtcgttcaa tgagaatgga taagaggctc gtgggattga cgattgtgag      60
   cggataacaa tatatttctg ggagcgaacg gagatataca tatggctagc atttcc         116
<210> 16
   <211> 11
   <212> PRT
   <213> Artificial Sequence
<220>
   <223> membrane translocation sequence
<400> 16
   Arg Arg Arg Arg Arg Arg Arg Arg Arg Arg Arg
   1               5                   10
<210> 17
   <211> 27
   <212> PRT
   <213> Artificial Sequence
<220>
   <223> membrane translocation sequence
<400> 17
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 18
   <211> 9
   <212> PRT
   <213> Artificial Sequence
<220>
   <223> membrane translocation sequence
<400> 18
   Arg Lys Lys Arg Arg Gln Arg Arg Arg
   1 5
<210> 19
   <211> 16
   <212> PRT
   <213> Artificial Sequence
<220>
   <223> membrane translocation sequence -
<400> 19
   Arg Gln Ile Lys Ile Trp Phe Gln Asn Arg Arg Met Lys Trp Lys Lys
   1 5 10 15
<210> 20
   <211> 16
   <212> PRT
   <213> Artificial Sequence
<220>
   <223> membrane translocation sequence
<400> 20
   Ala Ala Val Ala Leu Leu Pro Ala Val Leu Leu Ala Leu Leu Ala Pro
   1 5 10 15
<210> 21
   <211> 21
   <212> PRT
   <213> Artificial Sequence
<220>
   <223> membrane translocation sequence
<400> 21
   Lys Glu Thr Trp Trp Glu Thr Trp Trp Thr Glu Trp Ser Gln Pro Lys
   1               5                   10                  15
   Lys Lys Arg Lys Val
               20

## Description

### FIELD OF THE INVENTION

The present invention relates to plant biotechnology in general and more particularly to a process and vectors for plastid transformation of plants. Specifically, the present invention relates a process of genetic transformation of plant plastids, vectors for the process, and plants or plant cells obtained or obtainable according to the process of the invention. Moreover, the present invention relates to vectors conferring inducible gene expression in plant plastids, preferably by application of chemical inducers. The present invention also relates to a process of generating transgenic plants or plant cells transformed on their plastome having plastids, in which the expression of introduced genes can be induced, repressed or regulated by application of chemical substances or other external or internal stimuli.

### BACKGROUND OF THE INVENTION

Plastids and mitochondria contain their own DNA, DNA transcripts in the form of messenger RNA (mRNA), ribosomes, and at least some of the necessary tRNAs that are required for decoding of genetic information (Marechal-Drouard *et al.,* 1991). However, they are non-autonomous and depend on gene products encoded in the cell nucleus. Nevertheless, their genetic information is of sufficient complexity to make them an attractive target for gene technology. This is particularly the case with plastids, because plastids encode about 50% of the proteins required for their main function (photosynthesis) inside the plant cell. Plastids also encode their ribosomal RNAs, the majority of their tRNAs and ribosomal proteins. In total, the number of genes in the plastome is in the range of 120 (Palmer, 1991). The vast majority of proteins that are found in plastids are, however, imported from the nuclear/cytosolic genetic compartment.

With the development of general molecular cloning technologies, it became soon possible to genetically modify higher plants by transformation. The main emphasis in plant transformation was and still is on nuclear transformation, since the majority of genes, ca. 26.000 in the case of Arabidopsis thaliana, the complete sequence of which was recently published (The Arabidopsis Genome Initiative, 2000), is found in the cell's nucleus. Nuclear transformation was easier to achieve, since biological vectors such as Agrobacterium tumefaciens were available, which could be modified to efficiently enable nuclear transformation (Galvin, 1998). In addition, the nucleus is more directly accessible to foreign nucleic acids, while the organelles are surrounded by two envelope membranes that are, generally speaking, not permeable to macromolecules such as DNA.

A capability of transforming plastids is highly desirable since it could make use of the high gene dosage in these organelles that bears the potential of extremely high expression levels of transgenes. In addition, plastid transformation is attractive because plastid-encoded traits are not pollen transmissible; hence, potential risks of inadvertent transgene escape to wild relatives of transgenic plants are largely reduced. Other potential advantages of plastid transformation include the feasibility of simultaneous expression of multiple genes as a polycistronic unit and the elimination of positional effects and gene silencing that may result following nuclear transformation.

Methods that allow stable transformation of plastids could indeed be developed for higher plants. To date, two different methods are available, i.e. particle bombardment of tissues, in particular leaf tissues (Svab *et al.,* 1990), and treatment of protoplasts with polyethylene glycol (PEG) in the presence of suitable transformation vectors (Koop *et al.,* 1996). Both methods mediate the transfer of plasmid DNA across the two envelope membranes into the organelle's stroma. An overview of plastid transformation technology is given in Heifetz (2000) and Koop *et al.* (1996).

Plastid transformation methods usually rely on transformation vectors in which one or more transgenes are flanked by plastome sequences directing the insertion of the foreign genes by homologous recombination. Expression of the introduced gene or genes is achieved by placing the coding region under the control of regulatory elements. These regulatory elements usually contain a promoter active in plastids and operably linked to 5'- and 3'-untranslated regions. Promoters active in plastids can be obtained using plastome derived transcription activating sequences or using other sequences of synthetic or natural origin mediating transcription activity in the plastid. Examples for plastid promoters which mediate strong transcriptional activity are the rrn-promoter from the 16S-rRNA operon (Svab and Maliga, 1993) or the psbA promoter (Staub and Maliga, 1993). An example for a heterologous promoter is the phage T7 promoter which is, however, only active if the corresponding T7 RNA-polymerase is present. Alternatively, it is possible to insert foreign coding region(s) into a transcriptionally active site of the plastome, thus expressing the introduced genes by operable linkage to genes already present in the plastome (Staub and Maliga, 1995). In this case, it is necessary to fuse the coding region to be expressed to a sequence mediating, translation initiation, e.g. a ribosome binding site.

Transcript levels of the introduced gene(s) depend on promoter activity and turnover rates of the mRNA. In the case where the introduced gene(s) are controlled by plastid derived promoter elements, transcription patterns resemble the transcription patterns of the corresponding plastid genes. If an artificial operon has been generated by introducing the transgene(s) into a transcriptionally active site of the plastome, transcription activity is determined by the corresponding upstream promoter.

Plastid genes are actively transcribed in most cells, although there are development-, tissue-, or environment dependent variations in the intensity. In the green chloroplasts of photosynthetic tissue (e.g. leaves), a permanent strong transcriptional activity of the plastid genes can be observed, which is to a certain degree affected by the photosynthetic activity (e.g. day versus night or temperature condition). As a consequence, foreign genes introduced into the plastome are almost permanently transcribed. If the introduced gene(s) are operably linked to (a) sequence(s) mediating a strong translation activity, the expression level of the foreign gene may be extraordinarily high (Kuroda and Maliga, 2001). This is true at least for all green parts of the plant and reaches from the growth phase to the reproductive phase.

It is generally desired to achieve a high expression level of the introduced gene, e.g. in order to produce large quantities of pharmaceutical proteins in plants (such as somatotropin) or to generate plants with a high pest resistance level (such as B.t. cry protein). However, in many cases the permanent production of the recombinant gene product is undesirable or even detrimental. Permanent activity of the transgene can affect negatively the growth capacity or even plant health of crop plants by depriving metabolic energy for the production of the transgene.

Permanent activity of the transgene is even more adverse, if the resulting protein is toxic for the plastid or the plant cell. Proteins with slightly toxic effects on the plant may negatively affect normal development and biomass production of the plant. Also the expression of the desired recombinant protein may be negatively influenced. Toxic recombinant proteins may also prevent the plant from getting into the reproductive phase and thus prevent seed production. An example for harmful effects of recombinant gene products in plastids is the synthesis of polyhydroxybutyrate in tobacco, where significant contents of the product lead to growth reduction (Lössl *et al.,* 2003). If the protein product of an introduced gene is strongly toxic for the plastid or the plant cell, the generation of the transplastomic plant may be completely impossible.

Toxic effects caused by introduced genes can either result from a toxic effect of the protein itself or can result from an indirect effect of the protein on the metabolism. An example for an indirect effect is the case where the introduced gene codes for an enzyme which catalyses the production of toxic substances. Such detrimental effects of slightly toxic or strongly toxic proteins can be avoided if the introduced gene(s) are not permanently expressed, thus uncoupling the vegetative phase of the transgenic plant from the phase in which the transgene(s) are expressed in the fully developed plant.

Moreover, when considering aspects of biosafety, uncontrolled expression of transgenes in transplastomic plants can also be a problem with transgene products that do not exhibit negative effects on plant growth. Especially for gene products with unknown or potentially harmful effects on other organisms, permanent expression of these gene products in transplastomic plants during the whole growth period bears unpredictable risks. Therefore, a method to control the expression of such gene products is highly desirable. Consequently, control of transgene expression in plastids is an important factor in the production of transplastomic plants.

Up to now, there is only one example for induction of gene expression in plastids (US20020062502). The inventors made use of the T7 RNA polymerase / T7 promoter system (McBride *et al.,* 1994; US 5,925,806). A nucleus-encoded, plastid-targeted T7 RNA polymerase mediates transcription of a plastid-localized gene which is under the control of a T7 promoter. To make this system appropriate for inducible expression of the plastid transgene, the T7 polymerase gene was put under the control of a chemically inducible promoter (PR-1a promoter, benzothiadiazole induction). This system does, however, not provide a direct inducibility of plastid genes by external or internal factors. Instead, a signal is percepted in the nucleus and transmitted to the plastid via a protein intermediate. This system has, however, serious disadvantages. First, it relies on nuclear transformants which are capable of signal perception and transmission. The inducible plants cannot be generated by plastid transformation alone. Undesired spread of the nuclear transgene via pollen transfer, which is almost excluded for chloroplast transformants, is possible. Further, generation of two different transformants, nuclear transformants and plastid transformants, is time consuming. In order to generate plants transformed in both compartments, the nuclear transformant and the plastid transformant have to be hybridized, or both transformations have to be made subsequently. A method for regulation of transgenes in plastids, which relies on plastid transformation alone, would offer significant advantages.

Another problem when using standard plastid transformation methods arises from toxic effects of the genes to be expressed in the plastids on the bacteria used for cloning of the transformation vector: Plastid promoter and leader elements which are generally used to express the plastid transgenes are frequently also active in bacteria and lead to production of the corresponding protein in the cloning host. If this protein is not tolerated by the cloning host, transformation vector construction using standard molecular biology techniques is seriously impeded. Even genes for proteins with less toxic effects may be difficult to clone, if they are fused to regulatory elements which lead to an extraordinarily high expression level. A method wherein transgene expression in the cloning host is reduced or turned off would be highly desirable.

It is therefore an object of the present invention to provide a process of controlling expression of a plastome-encoded sequence of interest. A further object is to provide plants having a plastome-encoded sequence of interest, whereby expression of said sequence of interest can be controlled. It is another object of the invention to provide an efficient and highly versatile process of genetic transformation of plant plastids, which allows the production of substances in plastids which are toxic for the plastids or plants. It is another object of the invention to allow the production of transgenic plants which show normal plant health and growth capacity independent of the introduced gene(s). It is another object of the invention to minimize the risk of undesired uptake of substances produced in the transgenic plants and thus increase biosafety. It is another object of the invention to simplify cloning in bacteria of plastid transformation vectors containing sequences which are toxic for the bacteria.

### GENERAL DESCRIPTION OF THE INVENTION

The above objects are solved by a process of controlling expression of a plastome-encoded sequence of interest in a plant or in plant cells by externally applying to said plant or to said plant cells a chemical signal as a control signal,
wherein said control signal is adapted for an interaction of said chemical signal with the lac repressor as an intra-plastid component of the plastid protein expression machinery and
wherein expression of said sequence of interest is controlled by said interaction and by externally applying said control signal, as defined in claim 1. In the invention, said chemical signal is lactose or an analog thereof. Preferably, said intra-plastid component is an intra-plastid component of the plastid expression machinery of said sequence of interest but not of other plastid sequences.

The invention further provides a plant or plant cells capable of controlled expression of a plastome-encoded sequence of interest, said plant or plant cells comprising a plastome-encoded sequence of interest and having or encoding a heterologous intra-plastid component of the plastid protein expression machinery, said component being adapted for interacting with an externally provided chemical control signal such that expression of said sequence of interest can be controlled by said control signal as defined in claim 7. The plant of the invention comprises developing plants in all stages of development, including seeds. Also, parts of plants like leaves are comprised.

The invention further provides a process of producing a transplastomic plant or transplastomic plant cells transformed in their plastome with a sequence of interest, comprising transforming a plant or plant cells on their plastome with said sequence of interest operably linked to a lac operator and with a heterologous nucleotide sequence encoding an intra-plastid lac repressor.

The invention also provides a system for controlling expression of a sequence of interest in a transplastomic plant or in transplastomic plant cells, comprising the plant or plant cells of the invention and lactose or an analog thereof for controlling said expression in said plant or plant cells.

The inventors have found a general method of controlling expression of a plastome-encoded sequence of interest in plants or plant cells by an externally applied control signal. This general method is based on the interaction of said chemical or a physical signal with an intra-plastid component of the plastid protein expression machinery. Thus, the process of the invention does not require the generation of nuclear transformants of said plant or said plant cells. A basis of the invention is the surprising finding that chemical and physical signals, notably chemical signals, can be provided to plants or plant cells such that they reach and/or enter into plastids, whereby an interaction of said signal with an intra-plastid component of the plastid protein expression machinery is possible.

Plastids are surrounded by a two-membrane envelope and entry and exit of molecules is regulated. Plastids do not have large pores comparable to nuclear pores that allow almost unhindered passage of small molecules into and out of the nucleus. Further, externally provided physical or chemical signal have to cross the plasma membrane to get into the cytoplasm from where they can get into plastids. Moreover, the control signals have to cross the plant cell wall in order to be able to get into the cytoplasm and into the plastids. It was therefore very surprising to find that said physical or chemical signals of the invention can be provided to plant cells or plants such that they can accumulate in plastids in sufficient concentrations to allow controlling expression of a plastome-encoded sequence of interest.

The control process of the invention requires the generation of the transplastomic plant or plant cells according to the invention. This can be achieved by transforming a plant or plant cells on their plastome with said sequence of interest and a heterologous nucleotide sequence being or encoding said intra-plastid component of the plastid protein expression machinery. Said sequence of interest and said heterologous nucleotide sequence may be introduced into plastids of said plant or said plant cells on one type of recombinant nucleic acid (or vector) or on different recombinant nucleic acids, e.g. by co-transformation. Preferably, said sequence of interest and said heterologous nucleotide sequence are introduced with the same vector. Methods of plastid transformation are known in the art for several plant species. The invention can also be applied to plants for which plastid transformation becomes possible in the future. In plastid transformation, homologous recombination is typically used for introducing a desired sequence into a desired locus of the plastome. If said sequence of interest and said sequence being or encoding said intra-plastid component are introduced into the plastome with one vector, they may be present consecutively in said vector flanked by homologous flanks for homologous recombination with the plastome.

Regarding said sequence of interest, the invention is not limited. The sequence of interest may be a sequence native to the plastome of the plant to be transformed, which allows for example to control expression of a native plastid gene according to the invention. Preferably, however, the sequence of interest is heterologous to the plant or to the plant cells. The sequence of interest may be a sequence coding for a protein of interest to be expressed in said plant or in said plant cells. The sequence of interest may be introduced into plastomes as part of a recombinant nucleic acid that further contains a, preferably heterologous, transcription regulatory sequence (e.g. a promoter) operably linked to said sequence of interest. Said recombinant nucleic acid may further contain a, preferably heterologous, nucleotide sequence being or encoding an intra-plastid component of the plastid protein expression machinery. In one embodiment of the invention, said nucleotide sequence being or encoding an intra-plastid component is identical to said transcription regulatory sequence mentioned above. In another embodiment, said nucleotide sequence being or encoding an intra-plastid component is used in addition to said transcription regulatory sequence of said sequence of interest.

Said control signal of the invention is the handle that allows to control expression of said sequence of interest in said plant or said plant cells by the external application of said control signal. Said control signal is adapted for an interaction of said chemical signal with said intra-plastid component of the invention (see below). Said control signal is a chemical signal or a source of a chemical signal. Examples of physical signals as said control signal are light (notably a change in light intensity, a change in the dark-light cycle the plant or the plant cells are exposed to, a change in the spectral compositions of the light like the color of the light etc.) and a temperature change.

Said chemical signal or said source may be a proteinaceous chemical signal (i.e. a protein) or a non-proteinaceous (non-protein) chemical signal. Herein, a proteinaceous chemical signal is a signal that can be produced by expressing (transcribing and/or translating) a nucleic acid in an organism like a plant or in bacteria, i.e. a protein; a non-proteinaceous chemical signal cannot be produced by expressing a nucleic acid in an organism. Said chemical signal may be a high-molecular weight or a low-molecular weight chemical compound. Examples of high-molecular weight chemical compounds usable as a chemical signal are proteins (proteins used as externally applied signal are referred to as signal proteins in the following) or nucleic acids (nucleic acids used as externally applied nucleic acids are also referred to as nucleic acid signal herein). Examples of nucleic acid signals are DNA or RNA that can interfere (e.g. by RNA interference or by inducing a conformational change in a translation regulatory RNA operably linked to a transcript of said sequence of interest) or promote in plastids expression of said sequence of interest. Examples of low-molecular weight chemical compounds (which are preferably non-proteinaceous) are inducers like isopropyl thiogalactopyranosid (IPTG) or analogs thereof like lactose, tetracycline, arabinose, ethanol, steroids, copper ions or other inducers of inducible gene expression systems like those cited in US20020062502. Instead of using said inducers in a pure form, compositions containing said inducers may be applied externally to said plant or plant cell. An example of such a composition is whey that contains lactose. Whey is obtained as a by-product in cheese making and is a cheap source of lactose.

Externally applied chemical compounds may be subject to chemical reactions in plant cells, whereby said chemical signal may be produced in the cells from a source (or precursor) of said chemical signal. Examples of such chemical reactions are hydrolytic reactions of esters, amides, phosphates etc. by cellular hydrolytic enzymes, or trans-glycosylations (like the formation of 1,6-allolactose from lactose), phosphate group transfer reactions, or redox reactions. In such cases, the source of said chemical signal may be externally applied to said plant or plant cells. Said chemical signal capable of interacting with said intra-plastid component may then be produced from said source in plant cells.

Similarly, a proteinaceous chemical signal (e.g. said signal protein) may be produced in plant cells by expressing an externally applied nucleic acid encoding a signal protein. In this case, said nucleic acid is the source of said signal protein. Said signal protein is preferably provided with a plastid transit peptide for entering into plastids. Modes of external application of a signal protein or a source thereof to a plant or to plant cells are given below. If a nucleic acid is used as a source of a signal protein, said nucleic acid is externally applied to said plant or to said plant cells. In this embodiment, said externally applied nucleic acid is not adapted for integration in a nuclear chromosome; further, it is not adapted for inducing expression of said signal protein by external application of a small molecular chemical signal.

Being adapted for an interaction of said physical or chemical signal with said intra-plastid component comprises that said chemical or physical signal has to be able of reaching and entering into plastids after external application of said control signal to said plants or said plant cells. (Externally providing said chemical signal comprises externally applying said control signal and optionally forming said chemical signal from a source thereof.) Thus, the chemical or physical signal has to be able to transfer or to transmit over the plastidal double membrane, the cell membrane, the cell wall, and, preferably in the case of plants, also over the cuticula. The mode of application of said control signal to the plant or plant cells may support transfer of said chemical or physical signal into the plastids (see below). It was surprising to find that said control signals can be applied to plants or plant cells such that expression of a plastome-encoded sequence of interest can be controlled by externally applying said control signal.

The process of this invention does preferably not involve nuclear transformation. The process of the invention does not involve induction of expression of a nuclear encoded gene by said externally applied control signal.

Said intra-plastid component of the plastid protein expression machinery may be any component that is involved in plastid protein expression. Said intra-plastid component is adapted for interacting with said physical or said chemical signal, whereby expression of said sequence of interest can be controlled. Thus, said physical or said chemical signal and said component are pairs that are selected such that said interaction is possible. Examples of such signal/component-pairs are IPTG and the lac repressor; tetracycline and the tet repressor; N-(3-oxohexanoyl)-L-homoserine lactone and the LuxR transcriptional activator (cf. example 4); T7-polymerase and the T7-promoter. In any case, the interaction of said signal with said component has a functional effect on expression of said sequence of interest in plastids of said plant or plant cells, whereby said effect is absent when either said signal or said component is absent.

Preferably, said component is a nucleic acid or a protein. As a minimum requirement, said component is involved at least in plastid protein expression of said sequence of interest. Preferably, said component is not involved in plastid protein expression of other plastid sequences. Notably, said component is preferably not involved in expression of those native plastid sequences that are not sequences of interest. This means that said component is preferably required for controlling expression of said sequence of interest but has little or no influence on the expression of other plastid sequences. This may be achieved by using a heterologous intra-plastid component that is operably linked to said sequence of interest but not to other plastome sequences. In this way, said component may be provided such that exclusively expression of said sequence of interest is controlled.

However, the invention allows to control expression of two, three or more sequences of interest (e.g. for expressing multiple proteins of interest like multiple subunits of a multi-subunit protein of interest like an antibody). In this case, said component may be used for controlling all sequences of interest, whereby a single control signal may allow to control expression of all sequences of interest. Control of several sequences of interest can be easily achieved if said sequences are organized in an operon, whereby transcription of the operon may be controlled, or by providing each sequence of interest with the same regulatory control elements that respond to said externally applied control signal or to said component. Alternatively, each sequence of interest is operably linked to a different intra-plastid component, whereby expression of the various sequences of interest may be controlled independently by different externally applied control signals. The latter alternative may for example be used for growing a plant containing multiple sequences of interest (coding e.g. for different pharmaceutical or industrial proteins) up to a desired growth state, determining which of the encoded proteins is desired, followed by externally applying the signal for inducing expression of the determined sequence of interest.

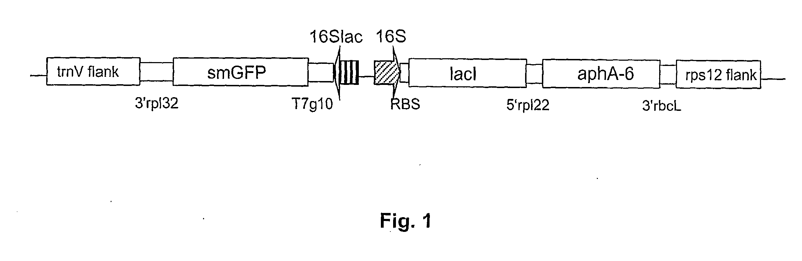

Said intra-plastid component may be a protein (e.g. a repressor, an activator, a transcription factor, factors involved in translation). Such proteins are referred to as regulatory proteins in the following. A regulatory protein may be encoded on a heterologous nucleotide sequence that is transformed into plastids of said plant or said plant cells preferably such that it is integrated into the plastome by homologous recombination. The heterologous nucleotide sequence encoding the regulatory .protein should contain operably linked regulatory elements for expressing said regulatory protein, like a promoter and 5' and 3' non-translated sequences. In some embodiments, the regulatory protein is constitutively expressed, e.g. if said regulatory protein is a repressor that represses expression of said sequence of interest. The plastids of said plant or said plant cell may be transformed with said heterologous nucleotide sequence encoding the regulatory protein independent from the transformation of said plastids with said sequence of interest. It is, however, generally more convenient to transform plastids of said plant or said plant cells simultaneously with said sequence of interest and with said heterologous nucleotide sequence encoding said intra-plastid component, for example by transforming with said recombinant nucleic acid that contains said sequence of interest and said heterologous nucleotide sequence (e.g. using a vector like pICF10501 shown in Fig. 1).

If said regulatory protein is used as said intra-plastid component, expression of said sequence of interest may depend on binding of said regulatory protein to a regulatory sequence element of said sequence of interest (e.g. to an operator). The binding affinity of said regulatory protein to said regulatory sequence should then be dependent on the interaction of said regulatory protein with said physical or chemical signal. As an example, said regulatory protein may be a repressor (e.g. lacl or tetR) and the binding affinity of the repressor to an operator is dependent on a small-molecular weight chemical signal (e.g. IPTG or tetracycline). Several known inducible protein expression systems may be adjusted for use in the present invention. Examples are heat-inducible (US 05187287) and cold-inducible (US05847102) systems, a copper-inducible system (Mett et al., 1993, Proc. Natl. Acad. Sci., 90, 4567-4571), steroid-inducible systems (Aoyama & Chua, 1997, Plant J., 11, 605-612; McNellis et al., 1998, Plant J., 14, 247-257; US06063985), an ethanol-inducible system (Caddick et al., 1997, Nature Biotech., 16, 177-180; WO09321334), and a tetracycline-inducible system (Weinmann et al., 1994, Plant J., 5, 559-569). A recently developed chemically inducible systems for plants uses a chimaeric promoter that can be switched on by the glucocorticoid dexamethasone and switched off by tetracycline (Bohner et al., 1999, Plant J., 19, 87-95). For a review on chemically inducible systems see: Zuo & Chua, (2000, Current Opin. Biotechnol., 11, 146-151). The inducible system for the present invention is the lac system based on the lac operon from E. coli. Prokaryotic inducible expression systems have never been used for achieving controlled expression of a plastome-encoded sequence of interest in plants.

If the lac system is used for the invention, said regulatory protein is the lac repressor that is preferably constitutively expressed. The lac repressor binds to the lac operator that is a regulatory sequence operably linked to said sequence of interest. The lac repressor binds to the lac operator in the absence of IPTG or an analog thereof like lactose. If IPTG or an analog thereof is externally applied as said chemical signal to the plant or to said plant cells, it can diffuse into plastids and interact with the lac repressor that is bound to the lac operator. The lac repressor having bound IPTG (or an analog thereof like lactose or 1,6-allolactose) dissociates from the operator, allowing transcription and expression of the sequence of interest.

Other preferred examples of said intra-plastid component of the invention are nucleic acids. Such nucleic acids may be regulatory elements that regulate expression of said sequence of interest, like a promoter. Preferably, said regulatory element as said component is a heterologous transcription regulatory sequence like a heterologous promoter that is operably linked to said sequence of interest. Said externally applied signal may then be a protein (in the following referred to as signal protein) that is capable of interacting with said transcription regulatory sequence. An example of a heterologous promoter as said intra-plastid component is the bacteriophage T7 promoter. Applying the T7 polymerase as said chemical signal into the plastids allows an interaction of the T7 polymerase with the T7 promoter and expression of said sequence of interest. Said regulatory element should be heterologous in order to ensure that the chemical signal controls exclusively expression of said sequence of interest but has little or no effect on expression of other plastid sequences.

In another embodiment wherein said intra-plastid component is a nucleic acid, said component may be or may be contained in a translation regulatory RNA. Said translation regulatory RNA may be an engineered 5'-untranslated region (5'-UTR) of an mRNA comprising a transcript of said sequence of interest; said chemical or physical signal may be capable of interacting with said 5'-UTR of said mRNA. Said 5'-UTR may e.g. comprise an RNA aptamer capable of regulating translation or termination of transcription of said mRNA. This type of regulation may function via alternative secondary structures of said RNA aptamer. Conformation changes of said mRNA may occur by binding of said chemical signal to the RNA aptamer of said mRNA. Said externally applied chemical signal is preferably a small-molecular weight chemical signal like flavin mononucleotide (Winkler et al. 2002) or theophylline (cf. example 6). Further, embodiments wherein said intra-plastid component is RNA are described below.

Said mRNA may comprise riboswitch elements (Winkler et al. 2002) capable of regulating translation or termination of transcription of said transcript. The modulation of translation or termination of transcription may function via alternative secondary structures of said riboswitch elements. The conformation chances of said mRNA may occur through binding of said chemical or physical signal by the mRNA.

Further, said intra-plastid component may be an enhancer or a silencer that is operably linked to said sequence of interest and said chemical or physical signal is capable of interacting with said enhancer or said silencer.

Said component of the invention is an intra-plastid component. "Intra-plastid" means that said component is or derives from a nucleic acid introduced into the plastome or a protein encoded in the plastome. Further, interaction of said component with said physical or chemical signal takes place predominantly inside of plastids. Interaction in plant cells outside of plastids does not achieve control of expression of said sequence of interest.

Modes of applying said signal protein or a source thereof are described next. If a protein is used as externally applied signal (signal protein), there are various possibilities regarding the mode of application to plants or plant cells. Such possibilties are described below, in PCT/EP03/13016, PCT/EP03/13018, PCT/EP03/13021 and references cited therein like Science (2000) 290, 979-982 and WO0189283. Instead of providing said signal protein directly to said plant or plant cells, an (externally applied) nucleic acid encoding said signal protein may be used as a source of said signal protein. Said nucleic acid may be applied such that expression of the signal protein is possible in cells of said plant. Said nucleic acid may be DNA or RNA. If the nucleic acid (signal) is DNA, transcription may take place in plastids. If said nucleic acid (signal) is RNA, translation of the signal protein may take place in the cytoplasm of cells of said plant. Preferably, said nucleic acid is an RNA viral vector or a DNA viral vector. Said viral vector should be infectious. The viral vector may be capable of amplification in cells of the plant, which allows strong expression of the signal protein. Preferably, the viral vector is further capable of cell-to-cell or systemic movement inside the plant, which allows controlling expression of said sequence of interest in cells that were not externally provided with said viral vector. For a review on the use of viral vectors see: Porta & Lomonossoff, 1996, Mol. Biotechnol., 5, 209-221; Yusibov et al., 1999, Curr.Top. Microbiol. Immunol., 240, 81-94). Further, the following documents describe systems based on DNA and RNA viral vectors: Kumagai et al., 1994, Proc. Natl. Acad. Sci. USA, 90, 427-430; Mallory et al., 2002, Nature Biotechnol. 20, 622-625; Mor et al., 2003, Biotechnol. Bioeng., 81; 430-437; US5316931; US5589367; US5866785; US5491076; US5977438; US5981236; WO0229068; WO02088369; WO02097080; WO9854342. Further, infectious copies of RNA viral vectors (Kumagai et al., 1995, Proc. Natl. Acad. Sci. USA, 92, 1679-1683) may be used. Among DNA and RNA viral vectors, RNA viral vectors (i.e. vectors that are derived from RNA viruses) are preferred. Preferred RNA viruses on which an RNA viral vector may be based are tobamoviruses, notably tobacco mosaic virus. If said signal protein is expressed in said plant cells but outside of plastids, the signal protein should preferably be provided with a plastid transit peptide that allows targeting said signal protein into plastids.

Methods how a nucleic acid can be applied to said plant or to said plant cells are generally known in the art. Preferred methods are Agrobacterium-mediated transformation or infiltration of leaves.

The control process of the invention may be used for inducing or suppressing expression of said sequence of interest, whereby inducing is preferred. Suppression of expression of a sequence of interest may e.g. be achieved using an operator (like the lac operator) operably linked to said sequence of interest, but no repressor is expressed in said plastids in the first place. When suppression is desired, the repressor may be externally provided to said plastids as a signal protein.

The control process may be carried out on intact plants or on plants after harvesting said plants. Further, the control process may be carried out on plant cells, e.g. in cell culture of plant cells.

The process of the invention can be carried out with any plant for which plastid transformation methods exist now or in the future. Preferred are higher crop plants. More preferred are dicot plants. Most preferred are Solanaceae and Brassicaceae. Plastid transformation protocols have been worked out at least for the following species: Tobacco (Svab Z, Maliga P, Proc Natl Acad Sci USA 90: 913-7 (1993)); Arabidopsis (Sikdar et al., Plant Cell Reports 18: 20-24 (1998)); Potato (Sidorov et al., Plant J 19: 209-216 (1999)); Tomato (Ruf et al., Nat Biotechnol 19, 870-5 (2001); Lequerella (Skarjinskaia et al., Transgenic Res. 12: 115-122 (2003)); Oilseed Rape (Hou et al., Transgenic Res. 12: 111-114 (2003)); Carrot (Kumar et al., Plant Physiol (2004)); and Rice (Khan et al., Nat Biotechnol 17: 910-5 (1999)).

### PREFERRED EMBODIMENTS

The a process of controlling expression of a plastome-encoded sequence of interest in a plant or in plant cells according to claim 1, wherein said lac repressor is not involved in expression of plastid sequences other than said sequence of interest.

Preferably, the process of controlling expression of said sequence of interest according to the invention is independent from transgenic elements of the nuclear genome.

### DEFINITIONS

- **3'-UTR:**: transcribed but not translated region of a (>) **gene,** downstream of a (>) **coding region;**
- **5'-UTR:**: transcribed but not translated region of a (>) **gene,** upstream of a (>) **coding region;** in (>) **plastid** (>) **genes,** the **5'-UTR** contains sequence information for translation initiation (ribosome binding site, (>) **RBS)** close to its 3' end;
- **aadA:**: (>) **coding region** of bacterial aminoglycoside adenyl transferase, a frequently used protein, that detoxifies antibiotic (>) **selection inhibitors** spectinomycin and/or streptomycin;
- **activator:**: protein which binds to an operator sequence and thereby activates transcription;
- **aphA-6:**: (>) **coding** region of bacterial aminoglycoside phosphotransferase A-6, a protein that detoxifies the antibiotic (>) **selection inhibitor** kanamycin;
- **chloroplast:**: (>) **plastid** containing chlorophyll;
- **coding region:**: nucleotide sequence containing the information for a) the amino acid sequence of a polypeptide or b) the nucleotides of a functional RNA; coding regions are optionally interrupted by one or more (>) intron(s);
- **flank, flanking region:**: DNA sequences at the 5' and 3' ends of inserts in a (>) **plastid** (>) **transformation vector,** which mediate integration into the target (>) plastome of sequences between the flanks by double reciprocal (>) **homologous recombination.** By the same mechanism, sequences can be modified or removed from the target (>) **plastome.** Thus, the flanks of the (>) **plastid** (>) **transformation vector** determine, where changes in the target (>) plastome are generated by (>) **transformation;**
- **gene expression:**: process turning sequence information into function; in (>) **genes** encoding polypeptides, gene expression requires the activity of a (>) **promoter,** which initiates and directs RNA polymerase activity, leading to the formation of a messenger RNA, which is subsequently translated into a polypeptide; in (>) **genes** encoding RNA, the (>) **promoter-mediated** activity of RNA polymerase generates the encoded RNA;
- **gene(s):**: nucleotide sequence(s) encoding all elements, which are required to secure function e.g. expression; genes are organised in (>) **operons,** which contain at least one complete (>) **coding region;** in (>) **genes** encoding polypeptides, these elements are: (1) a (>) promoter, (2) a 5' untranslated region ((>) **5'-UTR),** (3) a complete (>) **coding region,** (4) a 3' untranslated region ((>) **3'-UTR);** in (>) **genes** encoding RNA, the (>) **5'-UTR** and the (>) **3'-UTR** are missing; in (>) **operons** consisting of more than one (>) **coding region,** two subsequent complete (>) **coding regions** are separated by a (>) **spacer,** and (>) **promoter, (>) 5'-UTR,** and (>) **3'-UTR** elements are shared by the (>) **coding regions** of that (>)**operon;**
- **genome:**: Complete DNA sequence of a cell's nucleus or a cell organelle;
- **GFP:**: green fluorescent protein
- **homologous recombination:**: process leading to exchange, insertion or deletion of sequences due to the presence of (>) **flanks** with sufficient sequence homology to a target site in a (>) **genome;**
- **heterologous sequence:**: a sequence that does not occur in plastids, preferably in the entire (organellar and nuclear) genome, of the plant used in the process of the invention before plastids are transformed with said sequence.
- **intron:**: sequence interrupting a (>) **coding region;**
- **operator:**: nucleotide sequence which serves as a binding site for a regulatory protein;
- **operon:**: organisational structure of several (>) **genes** sharing a promoter;
- **plant(s):**: organism(s) that contain(s) (>) **plastids** in its (their) cells; plants may be multi-cellular or unicellular; this invention particularly relates to multicellular (>) **plants;** these include the group of gymnosperms (such as pine, spruce and fir etc.) and angiosperms (such as the *monocotyledonous* crops maize, wheat, barley, rice, rye, Triticale, sorghum, sugar cane, asparagus, garlic, palm tress etc., and non-crop monocots, and the *dicotyledonous* crops tobacco, potato, tomato, rape seed, sugar beet, squash, cucumber, melon, pepper, Citrus species, egg plant, grapes, sunflower, soybean, alfalfa, cotton etc.), and no-crop dicots as well as ferns, liverworths, mosses, and multicellular green, red and brown algae; examples of uni-cellular plants are *Chlamydomonas reinhardtii, Spirulina;*
- **plastid(s):**: organelle(s) with their own genetic machinery in (>) **plant** cells, occurring in various functionally and morphologically different forms, e.g. amyloplasts, (>) **chloroplasts,** chromoplasts, etioplasts, gerontoplasts, leukoplasts, proplastids etc;
- **plastome:**: complete DNA sequence of the (>) **plastid;**
- **promoter:**: nucleotide sequence functional in initiating and regulating transcription;
- **RBS, ribosomal binding site:**: DNA sequence element upstream of the (>) **translation start codon** of a (>) **coding region,** that mediates ribosome binding and translation initiation from the respective RNA transcript; **RBS** elements are either part of (>) **5'-UTRs** or of (>) **spacers;**
- **repressor:**: protein which binds to an operator sequence and thereby interferes with transcription;
- **RNA aptamer:**: RNA sequence the secondary structure of which may changel upon binding to a substrate;
- **selection inhibitor:**: chemical compound, that reduces growth and development of non-transformed cells or organelles stronger than that of transformed ones;
- **sequence of interest:**: modified or newly introduced sequence of any length: the purpose of a (>) **transformation** attempt; if introduction of a sequence is not intended, the length of the sequence of interest can be zero, i.e. it can be of interest not to have a sequence of interest;
- **termination:**: in the description of this invention, "termination" relates to discontinuation of transcription of RNA from a DNA sequence;
- **terminator:**: sequence element responsible for (->) **termination;**
- **transcription regulatory sequence:**: a DNA sequence involved in transcription of an operably linked sequence (e.g. of said sequence of interest);
- **transformation vector:**: cloned DNA molecule that was generated to mediate (>) **transformation** of a (>) **genome;**
- **transformation:**: process leading to the introduction, the excision or the modification of DNA sequences by treatment of (>) **plants** or plant cells including the use of at least one (>) **transformation vector;**
- **translation regulatory sequence:**: an RNA sequence involved in translation of an operably linked sequence (e.g. of said (transcribed) sequence of interest);
- **transgene:**: DNA sequence derived from one (>) **genome,** introduced into another one;
- **uidA:**: (>) **coding region** of bacterial β glucuronidase, a frequently used reporter protein.

### SHORT DESCRIPTION OF THE FIGURES

Figure 1 shows schematically the plastome insertion cassette of transformation vector pICF10501 containing two divergent transcription units: The tobacco plastid *rrn16* promoter (16S) controls transcription of the *lacl* and the *aphA-6* coding sequences, which are preceded by an artificial ribosome binding site (RBS) or the 5'-UTR of the tobacco plastid *rpl22* gene, respectively, and followed by the 3'-UTR of the *Chlamydomonas reinhardtii rbcL* gene. The smGFP coding sequence provided with the 5'-UTR of the bacteriophage T7 gene 10 and the 3'-UTR of the tobacco plastid *rpl32* gene, is transcribed from a modified *rrn16* promoter containing a lac operator sequence (16Slac). The insertion cassette is flanked by tobacco plastid DNA sequences for homologous recombination leading to insertion into the plastome between the *trnV* and the *3'rps12* genes.

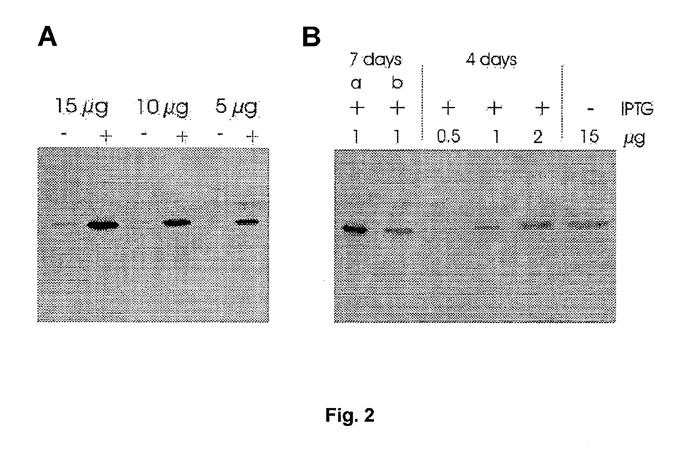

Figure 2: Immunological detection of GFP expression after induction with IPTG.
   A) Plastid transformant 557-1 containing the smGFP gene under the control of the 16S-lac3 promoter and the lacl gene (transformation vector pICF1050-1), was analyzed for its GFP content by Western blotting. For the uninduced sample (-), a young leaf of a ca. 7 cm high plant was removed before spraying the plant with a 1 mM IPTG solution. For the induced sample (+), the next youngest leaf was cut four days after spraying. The amount of total soluble protein loaded on the gel is given on the top of the lanes.
   B) Lower amounts of protein of the induced sample shown in A were loaded on the gel in order to allow quantification in comparison with the uninduced sample. In addition, protein from further leaves that were cut seven days after spraying was loaded. Leaf a was the next youngest leaf, leaf b an older leaf. Compared with a GFP standard, the amount of GFP in the first lane is in the range of 15 to 20 ng.

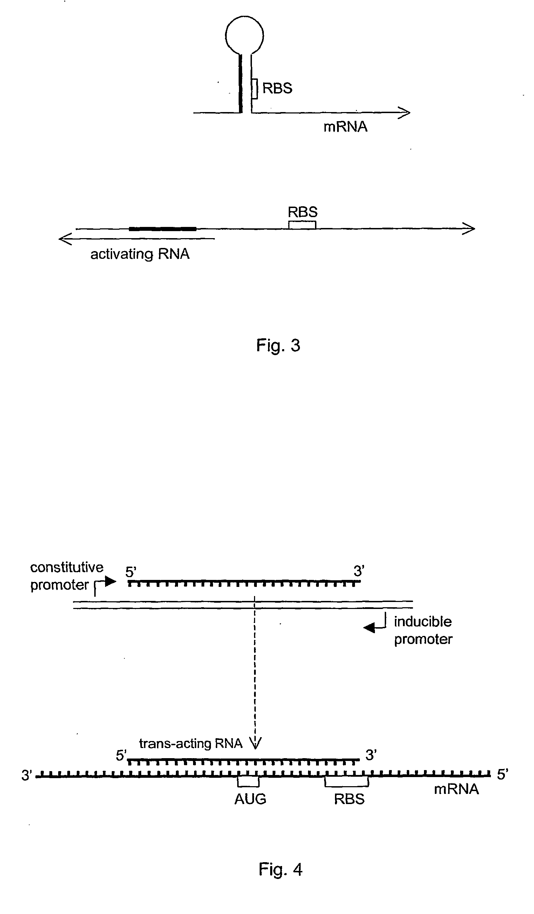

Figure 3 illustrates the principle of translational control used by Isaacs *et al.,* (2004) and in example 7. At the top, a 5'-UTR having self-complementarity leading to formation of a stem-loop structure is shown, whereby the ribosome binding site (RBS) is included in said stem-loop structure. The stem-loop structure prevents access of ribosomes to the ribosome binding site (RBS), whereby a sequence of interest encoded may said mRNA cannot be translated. A segment of said 5'-UTR (bold line) of the mRNA to be regulated has sequence complementarity to an activating RNA (a trans-acting RNA of the invention). In the presence of said activating RNA (bottom), said stem-loop resolved due to hybrid formation with the activating RNA, whereby said RBS is exposed for interaction with ribosomes, which allows translation initiation. The activating RNA is expressed from an inducible promoter.
Figure 4 gives a schematic representation of the principle of translational control used in example 8. The trans-acting RNA is transcribed from a constitutive promoter from double stranded plastid DNA (top). The trans-acting RNA has complementarity to a segment of a translation regulatory RNA of an mRNA and can form a hybrid shown at the bottom. The hybrid blocks the AUG codon and the RBS of the translation regulatory RNA, whereby translation is prevented. If transcription from the inducible promoter is induced by an externally applied control signal, an RNA complementary to said trans-acting RNA is transcribed, whereby said trans-acting RNA is scavenged by hybrid formation (not shown). As a result, the AUG codon and the RBS of the mRNA get exposed for ribosome binding. Alternatively, transcription of the trans-acting RNA may be controlled by a converse promoter which may be inducible.

### DETAILED DESCRIPTION OF THE INVENTION

This invention describes inter alia a process of controlling expression of a plastome-encoded sequence of interest in transplastomic plants. Control of gene expression may involve a regulatory molecule (notably a regulatory protein) which can bind to a regulatory element operably linked to said sequence of interest (e.g. located outside or within said sequence of interest). Examples for such a regulatory molecule are a repressor protein, binding of which prevents or impedes transcription of said sequence of interest, or an activator protein, binding of which enables or increases transcription of said sequence of interest. Other examples are proteins which bind to mRNA of said sequence of interest and regulate translation. A further example is an RNA polymerase which specifically transcribes said sequence of interest. Other examples for regulatory molecules are specific RNA splicing or processing factors. Furthermore, the regulatory molecule can also be a nucleic acid, e.g. an RNA molecule which prevents expression of the sequence of interest by interacting with its mRNA. Said regulatory molecule may itself be said control signal; alternatively, certain properties of said regulatory molecule may be changed in response to said externally applied signal, whereby expression of said sequence of interest is controlled.

In a general embodiment of this invention, binding of said regulatory molecule can be modified by said chemical signal or by said physical signal (such as temperature or light). Examples for such regulatory molecules properties of which can be changed by a chemical signal are repressor and activator proteins known from bacterial regulation systems as described below. When using such a control system, the regulatory protein can be encoded in the plastid and may be constitutively expressed, but certain properties (e.g. binding properties) can be changed in plastids by said externally applied signal, which allows controlling expression of the sequence of interest.

In a further embodiment of this invention, said regulatory molecule is said control signal. Control of expression of the sequence of interest can then be achieved by growing the plant or plant cells in the absence of the regulatory molecule and the regulatory molecule is externally applied to said plant or plant cells as said signal at a desired point in time. This can be achieved by direct external application of the regulatory molecule to the plant, or by externally applying a nucleic acid encoding said regulatory molecule, e.g. by infection with a modified plant virus (a viral vector).

Preferentially, the regulatory protein is derived from a prokaryotic organism. Numerous examples of regulatory proteins in prokaryotes have been described which can be used for controlling plastid gene expression as described in this invention. Examples for chemically regulatable repressor proteins from prokaryotic systems are the Lacl repressor from *Escherichia coli* or the TetR repressor from transposon tn10 (Hillen and Berens, 1994). In both cases, the promoter of the sequence of interest is provided with short sequence elements (operator elements) which are binding sites for the repressor protein. Binding of the repressor protein to the operator prevents transcription of the regulated sequence of interest. In the presence of an externally applied chemical inducer, the affinity of the repressor protein to the operator is reduced, so that transcription of the sequence of interest can proceed. Examples for chemical inducers are lactose or isopropyl-D-thiogalactopyranoside (IPTG) that interact with Lacl, tetracycline or anhydrotetracycline that interact with TetR, arabinose for the P_{BAD} promoter. In a preferred embodiment of this invention, the operator sequence is integrated into the plastome within or near the promoter of the sequence of interest to be controlled, and the repressor protein should also be encoded in the plastome such that it is constitutively expressed in a sufficient amount to prevent or impede expression of the sequence of interest. Expression can then be induced by the external application of said chemical signal acting as a chemical inducer at a desired time point.

A further way of controlling expression of said sequence of interest is by activator proteins. Examples of activator proteins in prokaryotic systems are the MalT activator from *Escherichia coli* (Schlegel *et al.,* 2002) or the LuxR activator from *Vibrio fischeri* (Dunlap, 1999). In contrast to repressor proteins, activator proteins do not prevent, but activate transcription when binding to operator sequences by interacting with the transcription machinery. Both activator proteins mentioned above require the presence of a chemical inducer as said chemical signal for exhibiting binding activity: MalT is activated by maltose, LuxR by N-(3-oxohexanoyl)-L-homoserine lactone. These substances can therefore also be used as chemical signals for inducing expression of said sequence of interest in plastids provided with appropriate regulation elements in a similar manner as described above for repressor proteins.

In a further embodiment of this invention, the described system can also be used to deactivate or decrease expression of a sequence of interest. This may be desired when the gene product of said sequence of interest is required during plant growth (e.g. herbicide resistance) but is undesired in the end product (e.g. in the harvested plant). Deactivation of expression may be achieved e.g. by growing of the plant in the presence of a chemical inducer which is left away when expression is not desired anymore. Further, expression of a repressor protein for the sequence of interest to be controlled may be activated.

Thus, applying said control signal or interrupting the application of said control signal may both be used for controlling expression of said sequence of interest.

The invention is not restricted to the control of a single sequence of interest. Operons containing several coding sequences may be also be controlled. If transcription of the operon is controlled, expression of all coding sequences can be regulated in the same way, which may be desired if several enzymes of a biosynthetic pathway are to be expressed. Alternatively, several coding sequences can be regulated separately, e.g. by using a combination of control systems responding to different chemical inducers.

### Translational control over the expression of the sequence of interest

Control over expression of a plastome-encoded sequence of interest in a plant or in plant cells may be achieved via translational control over said sequence of interest. A translational control can e.g. be achieved by regulating access of a ribosome binding site (RBS) to ribosomes. The access of the RBS to ribosomes can be altered by sequestering or exposing the RBS in a translation regulatory RNA (said translation regulatory RNA may be a 5'-UTR) operably linked to said sequence of interest. Sequestering or exposing the RBS may be achieved by regulating the secondary structure of said translation regulatory RNA near the RBS. Two basic principles may be used for regulating the secondary structure of said translation regulatory RNA. Both principles rely on reversible changes of RNA secondary structure (and optionally of the tertiary structure).

One principle is based on a translation regulatory RNA (e.g. 5'-UTR sequences) containing an RNA aptamer in the proximity of the RBS. Binding of a chemical control signal by said RNA-aptamer may modify the access of the RBS to ribosomes.

Another principle is based on sequestering or exposing the RBS on the 5'-UTR by using complementary repressor sequences. Said repressor sequences may be provided either on said translation regulatory RNA itself (in cis) or on a trans-acting RNA (in trans). In the case of cis-repression, the intra-molecular complementary sequences may form a stem-loop-structure which can be reversibly resolved by a trans-acting RNA (said trans-activating RNA described by Isaacs et al., 2004) by the formation of an alternative intermolecular hybrid. In the case of trans-repression, the intermolecular repressing RNA hybrid (hybrid of trans-acting RNA and a segment of the 5'-UTR) can be reversibly resolved by the formation of an alternative intermolecular hybrid with a further RNA molecule, or by suppressing the expression of the repressor. Thus, a trans-acting RNA may be used for suppressing translation or for activating translation.

Translation of said sequence of interest may be regulated by the use of riboswitch elements which are usually located in the 5'-UTR of the mRNA. Upon binding of a chemical signal to said riboswitch element, a conformational change may be induced in said mRNA, which can induce or repress translation activity by enabling or preventing access of the ribosome to the 5'-UTR. The binding of said chemical signal to the riboswitch element can either occur directly or indirectly. In the case of direct binding said signal molecule is bound by an RNA-element via an aptamer (Soukup and Breaker, 1999) of the 5'-UTR. In the case of indirect binding said signal molecule is bound via a protein factor.

### Translationally controlling expression of the sequence of interest by interaction of an externally applied chemical signal with a translation regulatory RNA (5'-UTR)

Said plant or said plant cells may contain in the plastid genome a recombinant nucleic acid, whereby said recombinant nucleic acid
- comprises said sequence of interest and
- codes for a translation regulatory RNA operably linked to said sequence of interest, said translation regulatory RNA being adapted for interaction with said chemical signal, whereby translation of said sequence of interest is controlled by said interaction. In this embodiment, said translation regulatory RNA is the intra-plastid component of the plastid protein expression machinery.
   Upon transcription from said recombinant nucleic acid, an mRNA transcript is formed containing said translation regulatory RNA and the transcribed sequence of interest. Said translation regulatory RNA may be a 5'-untranslated region (5'-UTR) and preferably contains elements required for translation of the sequence of interest. These are elements typically contained in a 5'-UTR of a plastid gene like a ribosome binding site (RBS). Said translation regulatory RNA is typically located upstream of said sequence of interest and is operably linked to said sequence of interest for translating said sequence of interest.
   Said translation regulatory RNA may further contain a segment adapted for interaction with an externally applied chemical signal, preferably a non-proteinaceous small-molecular chemical signal. Said segment may be an RNA aptamer being adapted for binding said chemical signal. In the absence of the chemical signal, the RNA aptamer may assume a secondary structure allowing access of the RBS to ribosomes, whereby translation of the sequence of interest is possible. In the presence of said chemical signal, said RNA aptamer binds said chemical signal, whereby the RNA aptamer assumes a secondary structure sequestering the RBS such that translation is not possible.
   RNA aptamers are RNA molecules the sequence of which is designed or selected such that tight and specific binding to a predetermined binding partner is possible. RNA aptamers have been identified that bind to proteins like enzymes (e.g. Rusconi et al., 2004, Jellinek et al., 1994) or to small-molecular binding partners like theophyllin (Suess et al., 2004), the FAD-cofactor (Roychowdhury-Saha et al., 2002), FMN (Winkler et al., 2002), or free adenine (Meli et al., 2002). For a review see Famulok and Mayer, 1999). RNA aptamers with high specificity for target molecules can be identified using the SELEX process (Tuerk and Gold, 1990).
   It was demonstrated in B. subtilis that translation of an mRNA sequence can be regulated by using a fusion of an aptamer to a downstream sequence containing a RBS (Suess et al. 2004). We found that translation of a recombinant plastid mRNA can be regulated by fusing an RNA aptamer (capable of binding to a predetermined externally applied control signal) with an artificial RBS. The interaction of the complex of said chemical signal and said RNA aptamer with the RBS may lead to a stimulation of translation. Alternatively, the interaction of the complex of said chemical signal and said RNA aptamer with the RBS may also lead to an inhibitory effect on translation.
   Controlling plastid gene expression by regulation of translation can either be applied independently or in combination with a regulation on the level of transcription. The latter has the advantage of enhancing the regulatory effect, which is preferred. In this preferred embodiment the externally applied chemical signal may interact with two or more different components of the plastid protein expression machinery in a synergistic mode. As an example, said chemical signal may interact with a regulatory protein (like the lac repressor) and an RNA aptamer. Interaction of said externally applied signal molecule with a regulatory protein may lead to an activation of transcription. Interaction of the same externally applied signal molecule an RNA aptamer may stimulate translation by allowing access of the previously sequestered RBS to ribosomes.
   Examples for externally applied chemical signals adapted for interaction with two or more different components of the plastid protein expression machinery on the level of translation include: lactose, lactose derivates, other carbon hydrates, tetracycline, antibiotics of various classes, herbicides, steroids, nutrients, proteins or sources thereof, nucleic acids or sources thereof.

### Translationally controlling expression of a sequence of interest by controlling the availability of a trans-acting RNA having complementarity to a segment of the translation regulatory RNA of said sequence of interest

Said plant or said plant cells may contain in the plastid genome a recombinant nucleic acid, whereby said recombinant nucleic acid
- comprises said sequence of interest and
- codes for a translation regulatory RNA operably linked to said sequence of interest, said translation regulatory RNA having a sequence segment complementary to a sequence segment of a trans-acting RNA, whereby the availability of said trans-acting RNA is controllable by an interaction of said control signal with an intra-plastid component of the plastid protein expression machinery.
   Said trans-acting RNA acts in trans with said translation regulatory RNA, whereby base-pairing is possible due to said complementarity. Said trans-acting RNA is expressed in plastids from a, preferably heterologous, sequence of the plastome. Said trans-acting RNA is preferably expressed independently from said sequence of interest. Unless stated differently, said translation regulatory RNA corresponds to that defined in the previous chapter.
   Said translation regulatory RNA may have a self-complementarity near its ribosome binding site (RBS), preferably a complementarity of the RBS, for enabling formation of a stem-loop structure involving said RBS, sequestering said RBS. Thereby, translation of said sequence of interest can be prevented in the absence of said trans-acting RNA. In the presence of said trans-acting RNA, said stem-loop structure in said translation regulatory RNA can be resolved by base-pairing between said trans-acting RNA and said complementary sequence segment of said translation regulatory RNA, leading to the exposure of the RBS and translation of the sequence of interest.
   Alternatively said translation regulatory RNA does not have a self-complementarity, but hybrid formation between a trans-acting RNA and a sequence segment of said translation regulatory RNA allows blocking of the RBS. In this embodiment, the presence of said trans-acting RNA suppresses translation of said sequence of interest. Scavenging of said trans-acting RNA by a further RNA having complementarity to said trans-acting RNA leads to an exposed RBS, whereby translation of said sequence of interest becomes possible. In this embodiment, control of said process is possible by interaction of an externally applied control signal with an intra-plastid component involved in transcription of said trans-acting RNA or of said further RNA.
   It has been shown that it is possible to control translation by the use of alternative secondary structures of RNA, which appear as a consequence of alternative, intramolecular or intermolecular, nucleic acid hybridizations (Isaacs et al., 2004, incorporated herein by reference). We have found that translation of a plastid mRNA can be regulated by externally applying to said plant or to said plant cells a control signal that controls the availability of a trans-acting RNA by an interaction with an intra-plastid component of the plastid translation machinery. Translation of said sequence of interest from its mRNA is in turn controlled by the interaction between said trans-acting RNA and said translation regulatory RNA that is operably linked to said sequence of interest. The source of said trans-acting RNA may also be a plastid transcription unit. Transcription of said trans-acting RNA may be controlled by externally applying to said plant or to said plant cells a control signal, wherein said control signal is adapted for an interaction with an intra-plastid component of the plastid gene expression machinery (e.g. the lactose/lac repressor/lac operator system).
   Plants or plant cells usable for this embodiment may be obtained by transforming the plastid genome with at least one construct containing an artificially engineered 5'-UTR operably linked to said sequence of interest, said 5'-UTR being capable of base-pairing and hybrid formation with said trans-acting RNA. The artificially engineered 5'-UTR for this embodiment must be capable of forming at least the following alternative secondary structures (i) and (ii) as a consequence of alternative hybrid formation.
   (i) formation, in said 5'-UTR, of an internal (intramolecular) hybrid like a stem-loop structure which leads to a blockage of the RBS, whereby interaction of the RBS with ribosomes is no longer possible.
   (ii) formation of an intermolecular hybrid between a trans-acting RNA and a sequence portion of said stem-loop structure. The intermolecular hybrid may release the previously sequestered RBS, whereby initiation of translation may become possible.

   For efficiently releasing the previously sequestered RBS, the intermolecular hybrid between the trans-acting RNA and the mRNA to be regulated should be stronger than the alternative intermolecular hybrid. Further, the sequence complementary to the RBS in the trans-acting RNA should be masked, since otherwise aberrant titration of ribosomes might occur. Masking of this sequence can be achieved by the use of an additional complementary sequence on said trans-acting RNA leading to the formation of a internal hybrid (see Isaacs et al., 2004). However, th internal hybrid in said trans-acting RNA should be weaker compared to the hybrid that can be formed with said mRNA to be regulated. Moreover the trans-acting RNA must be capable of resolving the intermolecular hybrid within the 5'-UTR of the mRNA to be regulated. This can be achieved by a short sequence element on the transacting RNA which is complementary to the loop structure within the mRNA to be regulated (Isaac et al., 2004).
   The above-described system for controlling translation of a sequence of interest can be applied in combination with a control on the level of transcription. The latter has the advantage of enhancing the regulatory effect. In a preferred embodiment, the externally applied signal molecule interacts with different components of the plastid gene expression machinery in a synergistic mode: e.g. a promoter and an RBS. Interaction of said externally applied signal with a promoter may lead to activation of transcription. Interaction of said externally applied signal molecule with the 5'-UTR leads to a stimulation of translation by releasing access of the previously sequestered ribosome binding site to the ribosome.
   The use of said trans-acting RNA as described herein can be applied to the simultaneous regulation of several operons. In this case, regulation of plastid transgene expression may be achieved by externally applying to said plant or to said plant cells a physical or chemical control signal or a source thereof, wherein said control signal is adapted for an interaction with an intra-plastid component of the plastid translation machinery. Said interaction may lead to the activation (or repression) of transcripts, which may simultaneously regulate other recombinant transcripts on the level of translation, yielding complex regulatory networks.
   In another embodiment of the invention (see example 8), the trans-acting RNA is made to inhibit translation of the mRNA to be regulated. The trans-acting RNA may be expressed from a constitutive promoter. Repression of the mRNA to be regulated may function by base pairing between a (antisense) sequence element of the trans-acting RNA and the RBS on the 5'-UTR of the mRNA to be regulated, thus preventing proper interaction of the RBS and the ribosome. Activation of translation may be achieved by expressing a second (sense) RNA which neutralizes the trans-acting RNA by hybrid formation. Binding between the trans-acting RNA and the second (sense) RNA should be stronger than binding between the trans-acting RNA and the RBS of the mRNA to be regulated. Alternatively, translation of the mRNA to be regulated can be achieved by suppressing the expression of the trans-acting RNA.
   Transcription of said sequence of interest and transcription of said trans-acting RNA may be controlled by the same or by different externally applied control signals. Said transacting RNA can be expressed from the same or from a different transcription unit than said sequence of interest. Expression from the same transcription unit can be used for boosting the regulatory effect of induction or repression. Expression from different transcription units can be used to construct artificial regulatory networks. Said trans-acting RNA may originate from a plastid transcription unit which is under the control of an externally applied chemical or an externally applied physical signal.

### The process and plants of the invention allows the production of substances which are toxic for the plants

Plastid transformation is often used as a method to produce substances, notably proteins of interest, in high amounts, making use of the high gene dosage in these organelles that bears the potential of extremely high expression levels of transgenes. There is a broad range of substances, from pharmaceutical proteins to technical enzymes, which can be produced in plastids. In addition, plastids may also be used for the production of non-proteinaceous substances like biopolymers by expression of enzymes which synthesize these substances from plastid metabolites. However, as plastids are essential for plant growth, the production of foreign substances frequently interferes with normal function of plastids. This invention offers a means to produce foreign substances which are harmful for plastids by suppressing or not activating their expression during plant growth. Expression of the product can be induced at a desired time point, e.g. shortly before plants are harvested. A preferred method for this invention is to control expression of the sequence of interest using a repressor protein responding to an induction signal, and a promoter containing an operator sequence. The sequence of interest may be introduced into the plastome downstream of said promoter containing the operator sequence. This promoter may be a modified plastid promoter, or a prokaryotic promoter containing the operator sequence. The operator sequence may be present within the promoter sequence or in its vicinity, e.g. at the start point of transcription, and several operator sites may be present. The sequence coding for the repressor protein is preferably inserted into the plastome in a different transcription unit so that it is constitutively expressed and is present in a sufficient amount to suppress transcription of the controlled sequence of interest by binding to the operator sequence. Expression of the controlled sequence of interest can be induced by providing the plant with said control signal which reduces binding of the repressor protein and therefore allows transcription. The control signal can be provided directly to the plant, e.g. by spraying, or indirectly, e.g. by providing a substance which is metabolized by the plant, whereby the induction signal is produced. As an alternative, the control signal can be an endogenous signal of the plant, e.g. when developmental changes such as fruit ripening are triggered. A regulatory protein influenced by such a developmental signal can be used for inducing or suppressing expression of a plastid sequence of interest at the time point of the developmental change.

Some gene products of said sequence of interest may be harmful to plants when they are produced in high amounts, while a low level of the product does not interfere with normal plant development. For these products, it may be sufficient to decrease transcription to a tolerable level; this can be achieved by using conventional repressor/operator systems like the lac repression system from E. coli, which usually allow a significant background expression. In general, it will be easier to obtain a high expression level after induction if a certain background expression level is tolerated. If tighter repression is required, i.e. for products which are harmful even in low amounts, different control systems can be combined, e.g. several operator sites can be inserted or several different repressor proteins can be used.

### The process and plants of the invention allow the production of transplastomic plants which show normal plant health and growth capacity

Even if the gene product of a sequence of interest is not toxic for the plants, it may be desirable to control expression of the sequence of interest. When a product is expressed in high amounts in plastids - which is desired in most cases - gene expression consumes a considerable amount of energy and metabolites, which may lead to a retarded plant growth. When expression of the sequence of interest is reduced to a limited level using the process described in this invention, plants can use their resources for normal growth and development.

### The process and plants of the invention allow the construction of vectors containing sequences which are toxic for bacteria

Vectors for plastid transformation are usually constructed in bacteria, mostly E. coli. Plastid regulatory elements are often recognized in bacteria such that a sequence of interest under the control of such regulatory elements can be expressed to some extent in the cloning host. This can lead to problems if a sequence of interest or its expression product is toxic to the cloning host, and in some cases the desired clones cannot be cloned in bacteria at all. The invention described herein allows to control expression in both plastids and the bacterial cloning host by using control elements in plastid transformation vectors that are also recognized in the cloning host. In this way, products toxic for E. coli can be cloned in E. coli in plastid expression vectors.

### Various mechanisms of regulation known from prokaryotic systems can be adapted for use in this invention

The gene expression machinery in plastids is in some respect similar to that of prokaryotic organisms: for example, translation is mediated by 70S ribosomes and transcription is mediated by E. coli-like and bacteriophage-like RNA polymerases. Therefore, regulation mechanisms for gene expression known from prokaryotic organisms may be used in plastids for expression control according to this invention by transferring the regulatory elements to plastids. An example of the invention is given below (example 1), wherein the lac repressor / operator system from E. coli was transferred to tobacco plastids: in example 1, the lac repressor is constitutively expressed from the tobacco plastome and impedes transcription of a plastome-encoded GFP gene which is under the control of a modifed promoter containing a lac operator site. Expression can be activated by treatment of the plant with the chemical inducer IPTG which binds to the lac repressor and reduces its affinity to the operator site. Similarly, corresponding regulation systems can be established in plastids with other repressor or activator systems known from prokaryotic organisms. Moreover, for creating more complex regulation systems in plastids, components from different systems can be combined, also with plastid-endogenous regulation elements. An example for this is the combination of a specific polymerase / promoter system, such as T7 polymerase / promoter system with a repressor system as described above. A further example is the generation of fusion proteins comprising DNA-binding and regulatory components derived from different regulation systems. With this approach, the original function of a regulatory protein can be changed, e.g. a protein originally functional as a repressor may be changed to a transcriptional activator by fusion with a suitable domain, as was done with the tet repressor for application in eukaryotic systems (reviewed in Berens and Hillen, 2003). Vice versa, a DNA binding protein originally active as a transcriptional activator may be changed to a repressor by placing its operator sequence into a promoter. As the above examples show, components from eukaryotic regulation systems can be used.

### Additional regulation can be achieved by applying the signal protein externally, e.g. via viral transfection

In this embodiment, the plant may be grown in the absence of the signal protein up to a desired growth stage. When induction or repression of the sequence of interest is desired, the signal protein may be externally applied as said control signal of the invention. One possibility of providing the signal protein is by viral transfection of the plant with a genetically modified virus encoding the signal protein, preferably in combination with a plastid targeting signal. Further possibilities include infiltration with other genetically modified vectors like Agrobacterium Ti plasmid, Agrobacterium - mediated protein delivery, or direct infiltration with a regulatory protein or a nucleic acid coding therefore. An advantage of this method of regulation is that the signal protein cannot cause unwanted effects on other plastid genes during plant growth. In addition, this method does not lead to stable integation of the externally applied nucleic acid encoding the signal protein into the plant hereditary material, which is advantageous for biological safety.

In analogy to the applications described above, repression of expression of a sequence of interest can be achieved by providing a repressor protein, while induction of a sequence of interest may be achieved by providing an activator protein. A more complex way for induction of a sequence of interest may be providing a repressor protein which prevents expression of a second repressor protein controlling transcription of said sequence of interest. For this application of the invention, regulatory proteins are preferably used that do not change their activity depending on external signals like inducers. Such regulatory proteins showing constitutive activity have been characterized as mutated or modified versions of many chemically regulated bacterial repressor or activator proteins. When using regulatory proteins with constitutive activity, application of a chemical inducer is not needed.

An example for this invention using external application of the signal protein is given in example 5. In this case, regulation is based on the interaction between the bacteriophage T7 RNA polymerase with a corresponding promoter. The sequence of interest (*uidA*) is plastome-encoded under the control of a specific T7 promoter such that the corresponding RNA polymerase is required for transcription. Sequence of interest expression can be induced by providing the T7 RNA polymerase as said chemical signal via inoculation of the plant or plant cells with a modified plant viral vector encoding the T7 polymerase. Expression of the T7 polymerase from the viral RNA may be mediated by an internal ribosome entry site (IRES) derived from a plant virus (crTMV) (Ivanov *et al.,* 1997, Skulachev *et al.,* 1999; WO9854342; WO0320927; WO0320928). The polymerase is preferably fused to a plastid targeting peptide so that it is imported into plastids where it can mediate transcription from the T7 promoter. In this example, the viral construct is integrated into a binary vector and can therefore be applied via infiltration of the transplastomic plants with *Agrobacterium* containing this vector. As stated above, further ways of applying the polymerase are possible. External application of the polymerase increases the tightness of the control compared to induction of a nuclear-encoded polymerase, as no expression in the uninduced state can occur. In addition, the sequence of interest cannot be transmitted via the pollen, which would be the case with a nuclear-encoded polymerase gene. Therefore, this induction method is advantageous for biosafety.

### External application of said signal protein from a cell-free composition (direct delivery)

Different methods can be used for the direct delivery of said signal protein (e.g. the T7 polymerase) into cells of said plant. Among the simplest ones is the direct delivery with the help of mechanical interaction with plant tissue. For example, microprojectile bombardment of polypeptide-coated particles can deliver said polypeptide into the plant cell. The protocol can be similar to those described for DNA delivery in plant transformation protocols (US 05100792; EP 00444882B1; EP 00434616B1). However, instead of DNA, said signal protein may be used for coating the particles. There is a description of a biolistic process that uses particle coating methods which are reasonably gentle for preserving the activity of said polypeptide (Sanford, Smith & Russell, 1993, Methods in Enzymol., 217, 483-509). In principle, other plant transformation methods can also be used e.g. microinjection (WO 09209696; WO 09400583A1; EP 175966B1), or liposome-mediated delivery (for review see: Fraley & Papahadiopoulos, 1982, Curr. Top Microbiol. Immunol., 96, 171-191).

Further, said externally applied signal protein may be applied from a cell-free composition to said plant or said plant cells. In this case, said signal protein preferably comprises a membrane translocation sequence (MTS) that enables entering of said signal protein into cells of said plant. Said MTS may be covalently or non-covalently bound to said signal protein. Preferably, it is covalently bound to said signal protein. Said MTS may be a peptide that endows said signal protein with the capability of crossing the plasma membrane of cells of said organism. Many such MTSs are known in the art. Frequently, they comprise several basic amino acids, notably arginines. The size of MTSs may vary largely, however, they may typically have 3 to 100 amino acids, preferably 5 to 60 amino acids. Typically, the MTS is included in the signal protein at its N-terminus. Said signal protein may be produced by standard protein expression techniques e.g. in E. coli. Purification of said signal protein after its expression is preferably done, notably removal of nucleic acids coding for said signal protein for biological safety. Said signal protein may be applied to a plant e.g. by spraying said plant with a liquid composition, preferably an aqueous solution, containing said signal protein. Preferably, measures are taken to facilitate entering of said signal protein into cells of said plant, notably measures that allow crossing of the plant cell wall and/or the outer plant layer. An example of such measures is slight wounding of parts of the plant surface e.g. by mechanical scratching. Another example is the use of cellulose-degrading enzymes in said cell-free composition to weaken or perforate the plant cell wall.

Many examples of MTSs, natural and synthetic, are known in the art. An MTS may be a simple amino acid repeat, for example a cationic peptide containing eleven arginines RRRRRRRRRRR (Matsushita et al., 2001, J. Neurosci., 21, 6000-6007). Another cationic MTS is a 27 amino acid long transportan (GWTLNSAGYL LGKINLKALA ALAKKIL) (Pooga et al., 1998, FASEB J., 12, 67-77). It is very likely that such peptides, for their penetration of the cell, exploit the asymmetry of the cellular plasma membrane where the lipid monolayer facing the cytoplasm contains anionic phospholipids (Buckland & Wilton, 2000, Biochim. Biophys. Acta/Mol. Cell. Biol. Of Lipids, 1483, 199-216). Many proteins contain subunits that enable their active translocation across the plasma membrane into cells. Examples of such subunits are the basic domain of HIV-1 Tat₄₉₋₅₇ (RKKRRQRRR) (Wender et al., 2000, Proc. Natl. Acad. Sci. USA, 97, 13003-13008), Antennapedia₄₃₋₅₈ (RQIKIWFQNR RMKWKK) (Derossi et al., 1994, J. Biol. Chem., 269, 10444-10450), the Kaposi Fibroblast Growth Factor MTS (AAVALLPAVL LALLAP) (Lin et al., 1995, J. Biol. Chem., 270, 14255-14258); the VP22 MTS (Bennet, Dulby & Guy, 2002, Nat. Biotechnol., 20, 20; Lai et al., 2000, Proc. Natl. Acad. Sci. U S A, 97, 11297-302); homeodomains from the *Drosophila melanogaster* Fushi-tarazu and Engrailed proteins (Han et al., 2000, Mol Cells 10, 728-732). It was shown that all these positively charged MTSs are able to achieve cell entry by themselves and as fusions with other proteins like GFP (Zhao et al., 2001, J. Immunol. Methods, 254, 137-145; Han et al., 2000, Mol Cells, 10, 728-732), Cre recombinase (Peitz et al., 2002, Proc. Natl. Acad. Sci. USA, 4489-4494) in an energy-independent manner. However, the fusion is not necessarily required for protein transport into the cell. A 21-residue peptide carrier Pep-1 was designed (KETWWETWWTEWSQPKKKRKV) which is able to form complexes by means of non-covalent hydrophobic interactions with different types of proteins, like GFP, b-Gal, or full-length specific antibodies. These complexes are able to efficiently penetrate cell membranes (Morris et al., 2001, Nature Biotechnol., 19, 1173-1176). The list of MTSs can be continued and, in general, any synthetic or naturally occurring arginine-rich peptide can provide the signal protein of the invention with the ability of entering plant cells (Futaki et al., 2001, J. Biol. Chem., 276, 5836-5840).

### External application of said signal protein using plant pathogens

Said signal protein may further be externally applied to said plant using a pathogenic microorganism that has a system for delivery of a protein like the signal protein of the invention into a host cell. Said signal protein may by expressably encoded in nucleic acids of said pathogenic microorganism, such that said signal protein can be delivered into a cell of said plant. A preferred example of such a pathogenic microorganism is a virulent or non-virulent *Agrobacterium,* whereby, for reasons of biological safety, said signal protein is preferably not encoded in the T-DNA of a Ti-plasmid of the *Agrobacterium* employed. Further examples of phytopathogenic microorganisms are Bordetella, Erwinia, Pseudomonas, Xanthomonas, Yersinia, the secretion systems of which may be used for the present invention. Examples for the use of the Yersinia type-III secretion system can be found in WO9952563.

Many plant and animal pathogenic bacteria use specialized secretion systems to deliver proteins into the host cells. Examples of such secretory systems are the type III secretion system of gram-negative bacteria (Binet et al., 1997, Gene, 192, 7-11; Thanassi & Hultgren, 2000, Curr. Opin. Cell Biol., 12, 420-430; Buttner & Bonas, 2002, Trends Microbiol., 10, 186-192; Buttner & Bonas, 2003, Curr. Opin. Plant Biol., 6, 321-319) and the type II secretory system of proteobacteria (Sandkwist, 2001, Mol. Microbiol., 40, 271-283). Multiple pathways of protein secretion from bacteria are described in the review of Thanassi and Hultgren (2000, Curr. Opin. Cell Biol., 12, 420-430. Type III secretion systems of different phytopathogenic bacteria can be used for delivering a protein into the plant cell. The Hrp gene cluster (type III protein secretion) was cloned from *Erwinia chrysanthemi* (Ham et al., 1998, Proc. Natl. Acad. Sci. USA, 95, 10206-10211); further examples are the *Pseudomonas syringae* secretion system (for review see Jin et al., 2003, Microbes Infect., 5, 301-310); and the secretory system of *Xanthomonas campestris* (Marois et al., 2002, Mol. Plant. Microbe Interact., 15, 637-646; Szurek et al., 2002, Mol. Microbiol., 46,13-23).

Plant pathogens (phytopathogens) should preferably be engineered such that they are able to transfer the protein of interest without causing severe ill effects on the host plant. Further, non-pathogenic bacteria can be engineered such that they have the part of the type III secretion system necessary for the delivery of a protein of interest into the plant cell, but not other parts that damage the host plant.

Among plant pathogens, agrobacteria are best suited for the present invention. Science (2000) 290, 979-982 and WO0189283 demonstrate the possibility of Agrobacterium-mediated transfer of Cre recombinase as a heterologous protein into host cells. The transfer was achieved by using a translational fusion of Cre with virulence proteins or their parts involved in protein translocation into the plant cell during contact with Agrobacterium. Cre recombinase delivery was not coupled with transfer of DNA encoding said recombinase, but was efficient enough to trigger recombination events in engineered target cells. The process of bacterium-mediated polypeptide delivery into plant cells requires the availability of engineered bacterial cells carrying the gene of said protein (WO0189283). Such a process is efficient enough to trigger selectable changes in plant cells in cell culture. Improvements of this methods are described in PCT/EP03/13016, PCT/EP03/13018, and PCT/EP03/13021.

### The processes and plants of the invention minimize the risk of undesired uptake of substances produced in the transgenic plants and thus increase biosafety

Production of recombinant proteins in transgenic plants offers substantial advantages compared to isolation of the proteins from natural sources or using fermentation technology. Pharmaceutical proteins isolated from animal or human material may be contaminated with co-purified pathogenic organisms, viruses, or prions. In contrast, plants are not known to harbour any pathogenic components on humans. Compared to fermentation technologies, protein production in plants is expected to be much more economical and can be easily scaled up using existing agricultural infrastructure. Plant protein production platforms allow the production of proteins for human or animal health, food additives, technical proteins or technical enzymes etc. However, large scale field production of transgenic plants bears an intrinsic risk of cross-contamination with agricultural products intended for the food chain. Possible reasons for a cross-contamination are mix-up of seeds, maintenance of transgenic plants from former vegetation periods in fields which are later used for non-GM plants (Fox, 2003), or undesired pollen transfer to non GM-crops grown in the vicinity.

It is a major task to implement consequent containment schemes for GM-crops, especially for those which contain substances which may be harmful if incorporated in an uncontrolled manner or which are not intended for consumption, over the whole production cycle. Preventing cross-pollination is one means for GM-crop containment. In addition, it would be desirable to have GM-plants which do not contain the recombinant product unless the expression of the product is induced.

The process of the present invention combines both requirements for safe protein production in transgenic plants. Using plastid transformants, pollen transfer of the sequence(s) of interest to wild-type crops is strongly reduced or excluded in most species, as plastids are mainly maternally inherited. All components of the system, which control plastid gene expression, are located in the plastome. This stands in contrast to the only other known method of inducing plastid gene expression which is based on the import of a nuclear encoded T7-polymerase. Moreover, using the process of this invention, it is possible to generate transplastomic plants which do not contain the recombinant protein of interest unless the production has been induced. Transgenic plants free of any potentially harmful proteins can be grown in the absence of the externally applied signal, thus preventing an undesired uptake of the substance by animals or humans. Expression can then be induced directly before harvesting or even after the harvest in a safe environment.

Using the present invention, undesired mix-up of GM-plants with non GM-crops either on the seed level or by accidental persistence of transgenic plants from former vegetation periods does not lead to any risks for human or animal health, as these plants do not come into contact with the externally applied inducing signal and therefore do not express the recombinant product(s).

This patent application claims priority of international patent application PCT/EP03/13656, filed December 3, 2003, which is incorporated herein by reference in its entirety.

### Example 1: Control of GFP expression in tobacco plastids using the lac repressor / operator system

### Construction of plastid transformation vector plCF10501

The *Iacl* coding sequence was PCR-amplified from E. coli strain XL1-Blue with primers olac1 (5'-gaccatggaaccagtaacgttatacgatg-3') and olac2 (5'-cactgcagtcactgcccgctttccag-3'), adding an Ncol and a Pstl restriction site to the ends. The coding sequence was fused to the plastid *rrn*16 promoter by insertion into the corresponding restriction sites of vector pKCZ (Zou *et al.,* 2003), replacing the *aadA* coding sequence, resulting in plasmid pICF9851. A modified version of the *rrn16* promoter containing a lac operator site between the -10 and-35 boxes was made by inverse PCR with primers olac3 (5'-acgattgtgagcggataacaatatatttctgggagcgaac-3') and olac4 (5'-caatcccacgagcctcttatc-3') from plasmid pICF7341 which contains the cloned promoter sequence amplified by PCR from tobacco DNA. The modified promoter was excised from the resulting plasmid with Sall and BamHI. A further fragment consisting of the smGFP coding sequence from pSMGFP4 (Davis and Vierstra 1998) flanked by the 5' untranslated sequence of the bacteriophage T7 gene10 and the 3' untranslated sequence of the plastid rpl32 gene (PCR-amplified from tobacco DNA) was excised from plasmid pICF9141 with BamHI and SaclI. Both fragments were ligated together into plasmid pICF9851 restricted with Xhol and SaclI so that two divergent transcription units (GFP controlled by the lac-modified *rrn16* promoter and *lacl* controlled by unmodified *rrn16* promoter) were obtained. A fragment containing these transcription units was excised with Sphl and Xhol and inserted after blunting of the overhanging ends with T4 DNA polymerase into the blunted Sdal restriction site of plasmid pICF9561, which contains the *aphA*-6 selection marker (Huang *et al.,* 2002) provided with plastid expression signals (5'-UTR of tobacco plastid rp/22, 3'-UTR of *Chlamydomonas reinhardtii rbcL),* and homologous flanks for recombination with the plastome (PCR-amplified from tobacco DNA). The plastome insertion site targeted with this vector (pICF10501) is between the *trnV(GAC)* and *3'rps12* genes of the tobacco plastome. A schematic depiction of the gene arrangement in pICF10501 is given in figure 1.

### Generation of tobacco plastid transformants

Tobacco seeds (Nicotiana tabacum cv. petite havana) were surface sterilized (1 min in 70% ethanol, 10 min in 5% Dimanin C, Bayer, Leverkusen, Germany), washed 3 times for 10 min in sterile H₂O and put on SCN-medium (Dovzhenko *et al.,* 1998). Plants were grown at 25°C in a 16h light/8h dark cycle (0.5 - 1 W/m², Osram L85W/25 Universal-White fluorescent lamps). Protoplast isolation was made according to Dovzhenko *et al.* (1998). Transformation using polyethyleneglycol (PEG) was performed as decribed in Koop *et al.* (1996), and alginate embedding according to Dovzhenko *et al.* (1998). After one week of culture in liquid medium, cells were transferred to agar-solidified RMOP-medium (Svab *et al.,* 1990) containing 25µg/ml kanamycin to select for transformants. Green regenerates were retrieved after 3-8 weeks and transferred to individual plates. In order to achieve homoplastomy, the individual lines were subjected to repeated cycles of shoot generation by cutting small leaf pieces which form new regenerates on RMOP-medium with 15µg/ml kanamycin. Rooting of selected regenerates was done on SCN-medium containing 15µg/ml spectinomycin. Plastid transformation was confirmed by molecular analysis of the regenerates (PCR and Southern analysis according to standard methods).

### Chemical induction of transgene expression

A transplastomic plant generated with pICF10501 was grown on SCN-medium to a height of ca. 7 cm. A young leaf (ca. 4 cm length) was cut and removed for analysis, and the plant was sprayed with ca. 1 ml of a 1 mM isopropyl-D-thiogalactopyranoside (IPTG) solution. After four and seven days, respectively, the next youngest leaf was cut off and also analyzed. Total soluble protein was extracted with 50 mM Na₂CO₃, pH9.6, 2 mM PMSF. Protein quantification was made according to the Bradford method with RotiQuant solution. Proteins were separated on a 15% polyacrylamide gel and transferred to nitrocellulose membrane, and GFP was detected immunologically using the ECL method (primary antibody directed against a recombinant protein corresponding to GFP from A. victoria; Santa Cruz Biotechnology). As shown in figure 2, the amount of GFP in the leaves had increased about 10-fold after four days, and a further increase could be observed seven days after spraying. The increase in GFP content corresponded to an increase in green fluorescence of leaves when irradiated with ultraviolet / blue light.

### Example 2: Plastid transformation of Solanum tuberosum using the lac repressor / operator system

In addition to tobacco, the gene control system described in this invention can also be used with other crop species such as potato (*Solanum tuberosum*)*.* This example illustrates efficient plastid transformation in potato following particle bombardment of protoplast-derived microcolonies using the vector described in Example 1. Due to the high degree of homology between the plastomes of tobacco and potato, the vectors containing tobacco flanking sequences can also be used for tobacco.

Plants of *S. tuberosum* cv. Walli were grown in vitro as sterile shoot cultures (20±1°C, 16h day, light intensity 75 ± 10 µmoles/m²/sec). New cultures were initiated every 2 months by transferring shoot tips (approx. 2 cm in length) to MS medium (Murashige and Skoog, 1962) in glass tubes (2.5 x 20 cm). Young fully expanded leaves are selected from 3-4 week old plants and used for protoplast isolation. Leaves are cut into 1 mm stripes with a scalpel and preplasmolysed in 10 ml of MMM-550 medium. MMM-550 medium contains 4.066 g/l MgCl₂6H₂O, 1.952 g/l 2-(N-morpholino) ethanesulfonic acid (MES) and -86 g/l mannitol (adjusted to 550 mOsm and pH 5.8). After 1 hour of incubation in the dark, the MMM-550 is removed and replaced with 10 ml of MMS-550 medium containing 0.4% w/v Macerozyme R10 and 0.4% Cellulase R10. MMS-550 medium contains 4.066 g/l MgCl₂6H₂O, 1.952 g/I MES and ~150 g/l sucrose (adjusted to 550 mOsm and pH 5.8). The leaf explants in enzyme solution are incubated for 16 hours in the dark at 25°C without shaking. The following day, the digestion is filtered through a 100 µm sieve into a centrifuge tube and then carefully overlaid with 2 ml of MMM-550 medium and centrifuged (10 min, 70 x g). Intact protoplasts are collected from the band at the interface and washed once by resuspending in 10 ml of potato protoplast culture medium followed by centrifugation (10 min, 50 x g). The protoplast culture medium contains 133.75 mg/l NH₄Cl, 950 mg/l KNO₃, 220 mg/l CaCl₂2H₂O, 185 mg/l MgSO₄7H₂O, 85 mg/l KH₂PO₄, B5 microelements (Gamborg *et al.* 1968), MS Fe-EDTA (Murashige and Skoog, 1962), 100 mg/l myo-inositol, 100 mg/l glutamine, 100 mg/l casein hydrolysate, 1 mg/l nicotinic acid, 10 mg/l thiamine hydrochloride, 1 mg/l pyridoxine hydrochloride, 250 mg/l xylose, 975 mg/l MES, 2 mg/l naphthalene acetic acid (NAA), 0.2 mg/l 2,4-dichlorophenoxyacetic acid (2,4-D), 0.5 mg/l 6-benzylaminopurine (BAP) and -94 g/l glucose (adjusted to 550 mOsm and pH 5.8). Protoplasts are counted and resuspended at 2x the required final plating density in protoplast culture medium (2.0 x 10⁵/ml) and mixed with an equal volume of 1.2% w/v alginic acid prepared in MMM-550 medium. Thin alginate layer culture in polypropylene grids is made as described in Dovzhenko *et al.* (1998). Following solidification of the alginate matrix, grids are cultured in 5cm Petri dishes containing 2 ml of protoplast culture medium. Protoplasts are incubated for one day in the dark (26±1°C) and then transferred to standard culture room conditions for further development (26±1°C, 16h day, light intensity 75 ± 10 µmoles/m²/sec).

12 to 15 days after embedding the grids containing potato microcolonies (approx. 8 cells) are transferred to 9 cm dishes containing SH-1 medium solidified with 0.4% w/v Gelrite. SH-1 medium contains 267.5 mg/l NH₄Cl, 1900 mg/l KNO₃, 440 mg/l CaCl₂2H₂O, 370 mg/l MgSO₄7H₂O, 170 mg/l KH₂PO₄, MS microelements and Fe-EDTA (Murashige and Skoog, 1962), Nitsch vitamins (Nitsch and Nitsch, 1969), 40mg/l adenine sulphate, 100 mg/l casein hydrolysate, 975 mg/l MES, 0.1 mg/l NAA, 0.5 mg/l BAP, 10 g/l sucrose and 50 g/I mannitol (adjusted to pH 5.8). Two days after plating on solid medium, protoplast-derived colonies are bombarded with aliquots of gold loaded with 25 µg of vector pICF10501 (see example 1) using the particle coating and bombardment conditions described in Mühlbauer *et al.* (2002). Selection of transformants is based on the resistance to the antibiotic kanamycin conferred by the *aphA-6* gene product. One day after bombardment, grids are transferred to dishes containing Gelrite-solidified SH-1 medium + 25 mg/l kanamycin and subcultured every 3 weeks to fresh selection dishes. Resistant colonies are transferred to 5 cm dishes containing SH-1 medium + 15 mg/l kanamycin. For regeneration, calli (approx. 5 mm in diameter) are transferred to SH-2 medium solidified with 0.4% w/v Gelrite containing 15 mg/l kanamycin. SH-2 medium is identical to SH-1 medium (see above) except that the NAA is replaced with 0.1 mg/l indole-3-acetic acid (IAA), BAP is replaced with 1 mg/l zeatin and the mannitol content is reduced from 50 g/I to 36 g/I. Shoots are removed from regenerating calli after 6-8 weeks of culture on SH-2 medium and transferred to antibiotic-free MS medium for rooting and further development. Analysis of transformants, induction with IPTG, and immunological determination of the GFP content is made as described in example 1.

### Example 3: Control of GFP expression in tobacco plastids using the tet repressor / operator system (Reference Example)

Transplastomic tobacco plants containing a recombinant GFP gene expression of which can be induced with tetracycline or anhydrotetracycline are generated by transformation with vector pICF10461. The general composition of plastid transformation vector pICF10461 corresponds to vector pICF10501 (described in example 1 and shown in figure 1), but instead of the *lacl* coding sequence the *tetR* coding sequence from transposon tn10 is inserted, and the modified *rrn16* promoter for the GFP gene contains a tet operator sequence instead of a lac operator. PCR-amplification of the tetR sequence from E. coli XL1-Blue is made with primers otet1 (5'-gaccatggctagattagataaaagtaaag-3') and otet2 (5'-cactgcagttaagacccactttcacatttaag-3'), and modification of the tobacco *rrn16* promoter by inverse PCR with primers otet3 (5'-acgtccctatcagtgatagagtatatttctgggagcgaac-3') and otet4 (5'-caatcccacgagcctcttatc-3'). The cloning procedure is made analogously to vector pICF10501, so that all other regulatory elements, selection marker, and plastome insertion site are identical.

Generation of tobacco plastid transformants is made as described in example 1. Induction of GFP expression is made by spraying plants with anhydrotetracycline solution (200 ng/ml). Immunological determination of the GFP content is made as described in example 1.

### Example 4: Control of GFP expression in tobacco plastids using the LuxR activator (Reference Example)

In this example, expression control of a recombinant GFP gene in tobacco plastids is mediated by the LuxR transcriptional activator protein from *Vibrio fischeri* (Dunlap, 1999). The GFP coding sequence is integrated into the plastome under the control of a modified *rrn16* promoter containing a 20 bp binding site for LuxR (lux-box) centred around nucleotide -42 from the transcription start. By this modification, the original -35 element and the sequence immediately upstream thereof which is essential for promoter function (Suzuki *et al.,* 2003), are destroyed, so that the promoter is not active in plastids. The *luxR* coding sequence is also integrated into the plastome in a divergent operon under the control of an unmodified *rrn16* promoter, conferring constitutive expression of the LuxR protein. For exhibiting transcriptional activator activity on the modified promoter, the LuxR protein requires the presence of a chemical inducer (VAI: N-(3-oxohexanoyl)-L-homoserine lactone). Thus, treatment of the transplastomic plants with the chemical inducer activates expression of GFP.

Construction of a plastid transformation vector is made in analogy to the construction of the transformation vector described in example 1, except that the *luxR* coding sequence is used instead of the lacl coding sequence, and the GFP coding sequence is put under the control of the modified *rrn16* promoter described in this example. Generation of tobacco plastid transformants is made as described in example 1. Induction of GFP expression is made by treatment of transplastomic plants with N-(3-oxohexanoyl)-L-homoserine lactone. Immunological determination of the GFP content is made as described in example 1.

### Example 5: Induction of a plastid transgene by a viral-delivered RNA polymerase (Reference Example)

### Generation of tobacco plastid transformants containing the uidA gene controlled by the T7 promoter

Vector pICF10571 contains a plastome insertion cassette containing two divergent transcription units: the *aadA* selection marker is controlled by the tobacco plastid *rrn16* promoter, an artificial ribosome binding site, and the 3'-UTR of the *Chlamydomonas reinhardtii rbcL* gene, and is therefore constitutively expressed in plastids, conferring resistance to spectinomycin and streptomycin. The *uidA* reporter gene coding for β-glucuronidase (GUS) is controlled by the promoter and 5'-UTR of bacteriophage T7. The transcription units are flanked by tobacco plastid DNA sequences for homologous recombination leading to insertion into the plastome between the *trnN* and *trnR* genes. Construction of the vector is made in the following way: a fragment containing the T7 promoter and 5'-UTR is excised from plasmid pET28a+ (Novagen) with BgIII and NcoI. A fragment consisting of the *uidA* coding sequence and the 3'UTR of the tobacco *rbcL* gene is excised from a plasmid described in Eibl *et al.* (1999) with Ncol and SacII. Both fragments are ligated into BgIII- and SacII - restricted transformation vector pKCZ (Zou *et al.,* 2003) which contains the selection marker and plastid DNA flanks. Generation of tobacco plastid transformants is made as described in example 1.

### Generation of a viral construct containing plastid targeted T7 RNA polymerase

Cloned cDNAs of the crucifer-infecting tobamovirus (cr-TMV; Dorokhov *et al.,* 1994) and of the turnip vein-clearing virus (TVCV; Lartey *et al.,* 1994) were obtained from Prof. Joseph Atabekov from Moscow University, Russia. A viral vector containing the T7 RNA polymerase coding sequence is made in several cloning steps. The resulting construct contains in sequential order: a 787 bp fragment from the Arabidopsis actin 2 promoter (ACT2, GenBank accession AB026654, bp 57962 to 58748), the 5' end of TVCV (GenBank accession BRU03387, bp 1 to 5455), a fragment of cr-TMV (GenBank accession Z29370, bp 5457 to 5677, with thymine 5606 changed to cytosine to remove the start codon of the coat protein) containing the sequence which acts as an internal ribosome entry site (Ivanov *et al.,* 1997, Skulachev *et al.,* 1999), a synthetic sequence encoding a peptide mediating protein import into plastids, the bacteriophage T7 RNA polymerase coding sequence (PCR-amplified from plasmid pACT7, Grachev and Pletnev, 1984), the cr-TMV 3' nontranslated region (GenBank accession Z29370, bp 6078 to 6312), and finally the nopaline synthase (Nos) terminator. The entire fragment is cloned between the T-DNA left and right borders of pICBV10, a CarbR pBIN19-derived binary vector. The resulting vector is transformed into Agrobacterium strain GV3101.

### Induction of GUS expression in transplastomic tobacco plants by agroinfiltration

Agroinfiltration of transplastomic plants with the construct containing the T7 RNA polymerase is made using the protocol of Yang *et al.* (2000). Protein extraction from leaf material and determination of GUS activity is performed as described in Jefferson *et al.* (1988).

### Example 6: Control of translation in tobacco plastids using a theophylline-binding aptamer (Reference Example)

In this example, expression of a recombinant GFP gene in tobacco plastids is controlled at the level of translation by using the theophylline-dependent aptamer described by Suess et al. (2004). A theophylline-binding RNA riboswitch is inserted into the 5'-untranslated region of the transgene and linked to the ribosomal binding site via a helical communication molecule for which a ligand-dependent slipping mechanism has been proposed (Suess et al. (2004), incorporated by reference herein). In the absence of the the chemical signal theophylline, a secondary structure is formed which inhibits ribosome binding to the 5'-UTR. Binding of theophylline induces a conformational change which makes the ribosomal binding site accessible.
The riboswitch described in Suess et al. (2004) (agatgataccagccgaaaggcccttggcagctctcg) is introduced into the 5' untranslated sequence of the bacteriophage T7 gene10 immediately upstream of the Shine-Dalgarno-sequence (AGGAG) via PCR: the sequence is included in a primer for amplification of a fragment containing of the smGFP coding sequence preceded by the Shine-Dalgarno-sequence (see example 1). A second PCR fragment consisting of the tobacco *rrn16* promoter and the rest of the T7 gene10 5'-untranslated region is amplified from plasmid plCF7341 (see example 1). Both fragments are joined via a BspMl restriction site and inserted into Sdal / AscI restricted plasmid pICF9561 (see example 1), which contains the *aphA-6* selection marker and homologous flanks for recombination with the plastome. Generation of tobacco plastid transformants is made as described in example 1. After integration into the plastome, an mRNA coding for smGFP and aphA-6 is constitutively transcribed from the recombinant run16 promoter, but translation of the GFP coding sequence is inhibited by the secondary structure in the 5'-UTR. Induction of GFP expression is made by treatment of transplastomic plants with theophylline. Immunological determination of the GFP content is made as described in example 1.

### Example 7: Simultaneous control of transcription and translation in tobacco plastids by lac control using an activating RNA

In this example, the chemical regulation of transcription described in example 1 is combined with a recently developed mechanism for regulating translation (Isaacs *et al.,* 2004), which consists in regulated expression of a small regulatory RNA molecule (said transacting RNA of the invention) which binds to a complementary sequence in the 5'-UTR upstream of the sequence of interest and hereby alters the secondary structure of the 5'-untranslated region in a way that translation is enabled. For establishing this regulation mechanism, translation of the sequence of interest is blocked by introducing a translation-inhibiting sequence in the 5'-untranslated region which is complementary to the sequence around the ribosome binding site and can therefore form a stem-loop which interferes with ribosome binding. In a different transcription unit, said transacting RNA that is complementary to the translation-inhibitory sequence is encoded. This small RNA specifically targets the inhibitory stem-loop, leading to a different structure wherein the ribosome binding site is exposed and translation is enabled. Both the sequence of interest and said transacting RNA are transcribed in plastids from lac-inducible promoters, so that addition of IPTG activates not only transcription of the sequence of interest but also its translation by stimulating expression of said transacting RNA.

A plastid transformation vector for introducing a GFP gene regulatable by this mechanism is constructed based on plastid transformation vector pICF10501 described in example 1. For replacing the T7 gene 10 5'-UTR upstream of the GFP coding sequence by the stem-loop forming 5'-UTR crR12 described in Isaacs *et al.,* 2004, the GFP coding sequence is PCR-amplified with this sequence added to the upstream primer oin1 (5'-tttggatccgaattctaccattcacctcttggatttgggtattaaagaggagaaggtatatgagtaaaggagaagaac -3') (downstream primer oin2: 5'- tatgagctcttatttgtatagttcatccatgcc -3') and inserted into pICF10501, partially restricted with BamHI and SacI. The additional transcription unit consisting of 16Slac-promoter and the small regulatory RNA (taR12 described in Isaacs *et al*., 2004) is added into the plasmid by insertion of a fragment made from synthetic oligonucleotides orn1 (5'- tttcggccgccgtcgttcaatgagaatggataagaggctcgtgggattgacgattgtgagc ggataacaatatatttctgggagcgaac -3') and orn2 (5'- tttcggccgtctagagatatatggtagtagtaagttaatttt cattaaccaccactaccaatcacctcctggatttgggtcgcccggagttcgctcccagaaatatattg -3') into the XmaIII restriction site downstream of the operon containing lac repressor and selection marker. Generation of tobacco plastid transformants, induction with IPTG, and immunological determination of the GFP content is made as described in example 1.

### Example 8: Simultaneous control of transcription and translation in tobacco plastids by lac control using an inhibitory RNA

Like in example 7, expression of GFP in transformed plastids is controlled by IPTG on .the level of transcription and translation. The mechanism for translational control is, however, different and is based on a translation-inhibiting small RNA molecule (said transacting RNA), which is complementary to part of the mRNA sequence of the gene of interest around the ribosomal binding site and start codon. This small RNA is expressed from a constitutive promoter and can hybridize to the mRNA, hereby preventing translation of mRNA transcribed from the uninduced lac promoter. A further lac-inducible promoter is placed downstream of the transcription unit for the translation-inhibiting small RNA in inverse orientation so that an antisense strand of the translation-inhibiting small RNA is transcribed. Induction with IPTG simultaneously activates transcription of the sequence of interest and allows its translation by suppressing the generation of the translation-inhibiting small RNA.

A plastid transformation vector for introducing a GFP gene regulatable by this mechanism is constructed based on plastid transformation vector pICF10501 described in example 1. A DNA fragment consisting of the tobacco chloroplast *rrn16* promoter, the DNA sequence for the inhibitory RNA, and the reverse 16SIac promoter, is produced by hybidizing and filling in oligonucleotides oan1 (5'-tttcggccgtcgttcaatgagaatggataagaggctcgtgggatt gacgtgagggggcag ggatggctatatttctgggagcgaacggaaatgctagccatatgtatatctcc -3') and oan2 (5'-tttcggccgccgtcgttcaatgagaatggataagaggctcgtgggattgacgattgtgagcggataacaatatatttctgggagcg aacggagatatacatatggctagcatttcc -3') and inserted into the XmaIII restriction site of pICF10501 downstream of the operon containing lac repressor and selection marker. Generation of tobacco plastid transformants, induction with IPTG, and immunological determination of the GFP content is made as described in example 1.

### REFERENCES

US5925806
US20020062502
WO9854342
Berens, C. and Hillen, W. (2003) Eur. J. Biochem. 270, 3109-3121
Davis, S.J. and Vierstra, R.D. (1998) Plant Mol. Biol. 36, 521-528
Dorokhov, Y.L., Ivanov, P.A., Novikov, V.K., Agranovsky, A.A, Morozov, S.Y., Efimov, V.A., Casper, R. and Atabekov, J.G. (1994) FEBS Lett. 350, 5-8
Dovzhenko, A., Bergen, U. and Koop, H.U. (1998) Protoplasma 204, 114-118
Dunlap, P.V. (1999) J. Mol. Microbiol. Biotechnol. 1, 5-12
Eibl, C., Zou, Z., Beck, a., Kim, M., Mullet, J. and Koop, H.U. (1999) Plant J., 19, 333-345
Famulok M, Mayer G Curr (1999) Top Microbiol Immunol 243:123-36
Fox, J.L. (2003) Nat. Biotechnol. 21, 3-4
Gamborg, O.L., Miller, R.A. and Ojima, K. (1968) Exp. Cell Res. 50, 151-158
Galvin S. B. (1998) Curr. Opin. Biotechnol. 9, 227-232
Grachev, M.A. and Pletnev,A.G. (1984) Bioorg. Khim. 10, 824-843
Gray M. W. (1991) in: Bogorad L. and Vasil I. K. (eds.), Cell Culture and Somatic Cell Genetics of Plants, Volume 7A, Academic Press, San Diego
Heifetz, P. (2000) Biochimie 82, 655-666
Hillen, W. and Berens, C. (1994) Annu. Rev. Microbiol. 48, 345-369
Huang, F.C., Klaus, S.M., Herz, S., Zou, Z., Koop, H.U. and Golds, T.J. (2002) Mol. Genet. Genomics, 268, 19-27
Isaacs, F.J., Dwyer, D.J., Ding, C., Pervouchine, D.D., Cantor, C.R., and Collins, J.J. (2004) Nature Biotechnology 22, 841-847
Ivanov, P.A., Karpova, O.V., Skulachev, M.V., Tomashevskaya, O.L., Rodionova, N.P., Dorokhov, Y.L. and Atabekov, J.G. (1997) Virology 232, 23-43
Jefferson, R.A., Kavanagh, T.A. and Bevan, M.W. (1987) Embo J., 6, 3901-3907
Jellinek D, Green LS, Bell C, Janjic N. (1994) Biochemistry 30:10450-6
Koop, H.U., Steinmüller, K., Wagner, H., Rossler, C., Eibl, C. and Sacher, L. (1996) Planta, 199, 193-201
Kuroda, H. and Maliga, P. (2001) Nucleic Acids Res. 29, 970-975
Lartey, R.T., Lane, L.C. and Melcher, U. (1994) Arch. Virol. 138, 287-298
Lössl, A., Eibl, C., Harloff, H.J., Jung, C. and Koop, H.U. (2003) Plant Cell Rep, 21, 891-899
Marechal-Drouard L., Kuntz M., Weil J. H. (1991) in: Bogorad L. and Vasil I. K. (eds.), Cell Culture and Somatic Cell Genetics of Plants, Volume 7A, Academic Press, San Diego
McBride, K.E., Schaaf, D.J., Daley, M. and Stalker, D.M. (1994) Proc. Natl Acad. Sci. USA 91, 7301-7305
Meli M, Vergne J, Decout JL, Maurel MC (2002) J Biol Chem 277:2104-11
Muhlbauer, S.K., Lössl, A., Tzekova, L., Zou, Z. and Koop, H.U. (2002) Plant J. 32, 175-184
Murashige, T. and Skoog, F. (1962) Physiol. Plant. 15, 473-497
Nitsch, J.P. and Nitsch, C. (1969) Science 169, 85
Palmer J. D. (1991) in: Bogorad L. and Vasil I. K. (eds.), Cell Culture and Somatic Cell Genetics of Plants, Volume 7A, Academic Press, San Diego
Roychowdhury-Saha M, Lato SM, Shank ED, Burke DH (2002) Biochemistry 26:2492-9
Rusconi CP, Roberts JD, Pitoc GA, Nimjee SM, White RR, Quick G, Scardino E, Fay WP, Sullenger BA (2004) Nat Biotechnol 22:1423-1428
Schlegel, A., Böhm, A., Lee, S.J., Peist, R., Decker, K. and Boos, W. (2002) J. Mol. Microbiol. Biotechnol. 4, 301-307
Skulachev, M.V., Ivanov, P.A., Karpova, O.V., Korpela, T., Rodionova, N.P., Dorokhov, Y.L., Atabekov, J.G. (1999) Virology 263, 139-154
Soukup G, Breaker R (1999) Proc Natl Acad Sci U S A 96:3584 - 3589
Staub, J.M. and Maliga, P. (1993) Embo J. 12, 601-606
Staub, J.M. and Maliga, P. (1995) Plant J. 7, 845-848
Suess, B., Fink, B., Berens, C., Stentz, R., and Hillen, W. (2004) Nucleic Acids Res. 32, 1610-1614
Svab, Z., Hajdukiewicz, P. and Maliga, P. (1990) Proc. Natl Acad. Sci. USA 87, 8526-8530
Svab, Z. and Maliga, P. (1993) Proc. Natl Acad. Sci. USA 90, 913-917
The Arabidopsis Genome Initiative (2000) Nature 408, 796-815
Tuerk C and Gold L (1990) Science 249:505 - 510
Winkler, Cohen-Chalamish, Breaker (2002) Proc Natl Acad Sci U S A. 99: 15908 - 15913
Yang, Y., Li, R., and Qi, M. (2000) Plant J. 22, 543-551
Zou, Z., Eibl, C. and Koop, H.U. (2003) Mol. Genet. Genomics, 269, 340-349

## Claims

1. A process of controlling expression of a plastome-encoded sequence of interest in a plant or in plant cells by externally applying to said plant or to said plant cells a control signal selected from lactose or an analog of lactose,
wherein said plant or said plant cells contain in the plastid genome a recombinant nucleic acid comprising said sequence of interest and, operably linked thereto, a lac operator, said plant or said plant cells further having or encoding an intra-plastid lac repressor.

2. The process according to claim 1, wherein said lac repressor is encoded by said recombinant nucleic acid or by a further recombinant nucleic acid integrated into said plastid genome.

3. The process according to claim 1 or 2, wherein said controlling is inducing expression of said sequence of interest.

4. The process according to any one of claims 1 to 3, wherein said controlling is suppressing expression of said sequence of interest.

5. The process according to any one of claims 1 to 4, wherein said process is carried out on an intact plant or after harvesting said plant or said plant cells.

6. The process according to any one of claims 1 to 5, wherein said lactose analog is isopropyl thiogalactopyranoside (IPTG).

7. Plant or plant cells capable of controlled expression of a plastome-encoded sequence of interest, said plant or plant cells comprising in the plastid genome a recombinant nucleic acid comprising said sequence of interest operably linked to a lac operator, said plant or said plant cells further having or encoding an intra-plastid lac repressor.

8. Said plant or plant cells according to claim 7, wherein said lac repressor is encoded by a heterologous nucleotide sequence transformed into plastids of said plant or said plant cells.

9. Said plant or plant cells according to claim 7 or 8, wherein said lac repressor is constitutively expressed in said plant or said plant cells.

10. A process of producing a plant or plant cells transformed on their plastid genome with a sequence of interest, comprising transforming a plant or plant cells on their plastome with said sequence of interest operably linked to a lac operator and with a heterologous nucleotide sequence encoding an intra-plastid lac repressor.

11. A system for controlling expression of a sequence of interest in a transplastomic plant or in transplastomic plant cells, comprising the plant or plant cells according to claim 7 and lactose or an analog of lactose for controlling expression of said sequence of interest in said plant or plant cells.

12. Said system according to claim 11, wherein said lactose analog is isopropyl thiogalactopyranoside (IPTG).

## Patentansprüche

1. Verfahren zur Kontrolle der Expression einer plastomkodierten gewünschten Sequenz in einer Pflanze oder in Pflanzenzellen durch externe Applikation auf die Pflanze oder die Pflanzenzellen eines Kontrollsignals ausgewählt aus Lactose oder einem Lactoseanalogon, wobei die Pflanze oder die Pflanzenzellen im Plastidengenom eine rekombinante Nukleinsäure enthalten, umfassend die gewünschte Sequenz und, operativ damit verbunden, einen Lac-Operator, wobei die Pflanze oder die Pflanzenzellen ferner einen intraplastidären Lac-Repressor enthalten oder dafür kodieren.

2. Das Verfahren gemäß Anspruch 1, wobei der Lac-Repressor von der rekombinanten Nukleinsäure oder von einer weiteren rekombinanten Nukleinsäure, die in das Plastidengenom integriert ist, kodiert wird.

3. Das Verfahren gemäß Anspruch 1 oder 2, wobei die Kontrolle Induktion der Expression der gewünschten Sequenz ist.

4. Das Verfahren gemäß einem der Ansprüche 1 bis 3, worin die Kontrolle Unterdrücken der Expression der gewünschten Sequenz ist.

5. Das Verfahren gemäß einem der Ansprüche 1 bis 4, worin das Verfahren auf einer intakten Pflanze oder nach dem Ernten der Pflanze oder Pflanzenzellen durchgeführt wird.

6. Das Verfahren gemäß einem der Ansprüche 1 bis 5, wobei das Lactoseanalogon Isopropylthiogalactopyranosid (IPTG) ist.

7. Pflanze oder Pflanzenzellen, die zur kontrollierten Expression einer Plastomkodierten gewünschten Sequenz befähigt ist/sind, wobei die Pflanze oder die Pflanzenzellen im Plastidengenom eine rekombinante Nukleinsäure enthalten, die die gewünschte Sequenz und, operativ damit verbunden, einen Lac-Operator umfassen, wobei die Pflanze oder die Pflanzenzellen ferner einen intraplastidären Lac-Represssor aufweisen oder für einen solchen kodieren.

8. Die Pflanze oder die Pflanzenzellen gemäß Anspruch 7, worin der Lac-Repressor von einer heterologen Nukleotidsequenz kodiert wird, die in die Plastiden der Pflanze oder der Pflanzenzellen transformiert ist.

9. Die Pflanze oder die Pflanzenzellen gemäß Anspruch 7 oder 8, wobei der Lac-Repressor in der Pflanze oder den Pflanzenzellen konstitutiv exprimiert wird.

10. Verfahren zur Herstellung einer Pflanze oder von Pflanzenzellen, die auf ihrem Plastidengenom mit einer gewünschten Sequenz transformiert sind, umfassend das Transformieren einer Pflanze oder von Pflanzenzellen auf ihrem Plastom mit der gewünschten Sequenz, die operativ mit einem Lak-Operator gekoppelt ist, und mit einer heterologen Nukleotidsequenz, die für einen intraplastidären Lac-Repressor kodiert.

11. System zur Kontrolle der Expression einer gewünschten Sequenz in einer transplastomischen Pflanze oder in transplastomischen Pflanzenzellen, umfassend die Pflanze oder die Pflanzenzellen gemäß Anspruch 7 und Lactose oder ein Lactoseanalogon zur Kontrolle der Expression der gewünschten Sequenz in der Pflanze oder den Pflanzenzellen.

12. Das System gemäß Anspruch 11, wobei das Lactoseanalogon Isopropylthiogalactopyranosid (IPTG) ist.

## Revendications

1. Procédé pour contrôler l'expression d'une séquence codée par un plastome, à laquelle on s'intéresse, dans une plante ou dans des cellules végétales, par application externe à ladite plante ou auxdites cellules végétales d'un signal de contrôle choisi parmi le lactose et un analogue de lactose,
dans lequel ladite plante ou lesdites cellules végétales contiennent, dans le génome de plastide, un acide nucléique recombiné comprenant ladite séquence à laquelle on s'intéresse et, lié à celle-ci de façon fonctionnelle, un opérateur lac, ladite plante ou lesdites cellules végétales ayant ou codant en outre un répresseur lac intra-plastidique.

2. Procédé selon la revendication 1, dans lequel ledit répresseur lac est codé par ledit acide nucléique recombiné ou par un autre acide nucléique recombiné intégré dans ledit génome de plastide.

3. Procédé selon la revendication 1 ou 2, dans lequel ledit contrôle induit l'expression de ladite séquence à laquelle on s'intéresse.

4. Procédé selon l'une quelconque des revendications 1 à 3, dans lequel ledit contrôle est une suppression de l'expression de ladite séquence à laquelle on s'intéresse.

5. Procédé selon l'une quelconque des revendications 1 à 4, lequel procédé est mis en oeuvre sur une plante intacte ou après récolte de ladite plante ou desdites cellules végétales.

6. Procédé selon l'une quelconque des revendications 1 à 5, dans lequel ledit analogue de lactose est l'isopropylthiogalactopyranoside (IPTG).

7. Plante ou cellules végétales capables d'une expression contrôlée d'une séquence codée par un plastome à laquelle on s'intéresse, ladite plante ou lesdites cellules végétales comprenant, dans le génome de plastide, un acide nucléique recombiné comprenant ladite séquence à laquelle on s'intéresse liée de façon fonctionnelle à un opérateur lac, ladite plante ou lesdites cellules végétales ayant ou codant en outre un répresseur lac intra-plastidique.

8. Plante ou cellules végétales selon la revendication 7, dans lesquelles ledit répresseur lac est codé par une séquence de nucléotides hétérologue transformée dans des plastides de ladite plante ou desdites cellules végétales.

9. Plante ou cellules végétales selon la revendication 7 ou 8, dans lesquelles ledit répresseur lac est exprimé de manière constitutive dans ladite plante ou lesdites cellules végétales.

10. Procédé pour produire une plante ou des cellules végétales, transformées au niveau de leur génome de plastide par une séquence à laquelle on s'intéresse, comprenant la transformation d'une plante ou de cellules végétales au niveau de leur plastome avec ladite séquence à laquelle on s'intéresse liée de façon fonctionnelle à un opérateur lac et avec une séquence de nucléotides hétérologue codant un répresseur lac intra-plastidique.

11. Système pour contrôler l'expression d'une séquence à laquelle on s'intéresse dans une plante transplastomique ou des cellules végétales trans-plastomiques, comprenant la plante ou les cellules végétales selon la revendication 7 et du lactose ou un analogue de lactose pour contrôler l'expression de ladite séquence à laquelle on s'intéresse dans ladite plante ou lesdites cellules végétales.

12. Système selon la revendication 11, dans lequel ledit analogue de lactose est l'isopropylthiogalactopyranoside (IPTG).
